# EUROPÄISCHE PATENTSCHRIFT

(11) **EP 1 280 893 B1**
(45) Veröffentlichungstag und Bekanntmachung des Hinweises auf die Patenterteilung: **21.07.2010**
(21) Anmeldenummer: 01938205.0
(22) Anmeldetag: 08.05.2001
(51) Int. Cl.: C12N 15/81, C12R 1/645

(54) **VERFAHREN ZUM HERSTELLEN VON PROTEINEN IN EINER HEFE DER GATTUNG ARXULA UND DAFÜR GEEIGNETE PROMOTOREN**
METHOD FOR THE PRODUCTION OF PROTEINS IN A YEAST OF THE GENUS ARXULA AND SUITABLE PROMOTERS
PROCEDE DE PRODUCTION DE PROTEINES DANS UNE LEVURE DU GENRE ARXULA ET PROMOTEURS ADAPTES A CETTE FIN

(30) Priorität: 08.05.2000 DE 10022334
(43) Veröffentlichungstag der Anmeldung: 05.02.2003
(73) Patentinhaber: Artes Biotechnologie GmbH, 40764 Langenfeld (DE); Institut für Pflanzengenetik und Kulturpflanzenforschung (IPK), 06466 Gatersleben (DE)
(72) Erfinder: KUNZE, Gotthard, 06466 Gatersleben (DE); WALTER, Ines, 91349 Egloffstein (DE); RÖSEL, Harald, 04157 Leipzig (DE); BÖER, Erik, 38895 Langenstein (DE); STOLTENBURG, Regina, 04157 Leipzig (DE); WARTMANN, Thomas, 06408 Aderstedt (DE); DUC, Bui, Minh, A-1030 Wien (AT)
(74) Vertreter: Herzog, Martin
(86) Internationale Anmeldenummer: PCT/EP2001/005225
(87) Internationale Veröffentlichungsnummer: WO 2001/085925

(56) Entgegenhaltungen:
- DD-A- 298 821
- DE-A- 4 425 058
- ROESEL H ET AL: "INTEGRATIVE TRANSFORMATION OF THE DIMORPHIC YEAST ARXULA ADENINIVORANS LS3 BASED ON HYGROMYCIN B RESISTANCE" CURRENT GENETICS, NEW YORK, NY, US, Bd. 33, Februar 1998 (1998-02), Seiten 157-163, XP000994832 ISSN: 0172-8083
- DATABASE EMBL/GENBANK/DDBJ [Online] EBI; 4. April 1996 (1996-04-04) "S. cerevisiae DNA region from chromosome XIV, left arm" XP002185260
- DATABASE EMBL/GENBANK/DDBJ [Online] EBI; 21. November 1996 (1996-11-21) "C. albicans FET3 gene" XP002185261
- DATABASE EMBL/GENBANK/DDBJ [Online] EBI; 4. August 1999 (1999-08-04) "Homo sapiens chromosome 19 clone CTB-186G2, complete sequence" XP002185262
- DATABASE EMBL/GENBANK/DDBJ [Online] EBI; 18. November 1999 (1999-11-18) "Mus musculus clone RP23-317K23" XP002185263
- DATABASE EMBL/GENBANK/DDBJ [Online] EBI; 10. Mai 2000 (2000-05-10) "Arxula deninivorans afet3 gene for ferro-O2-oxidoreductase" XP002185264
- DATABASE EMBL/GEN BANK/DDBJ [Online] EBI; Arxula adeninivorans ARFC3 gene, 1998 XP002177392
- WARTMANN T ET AL: "AIL V1 GENE FROM THE YEAST ARXULA ADENINIVORANS LS3 - A NEW SELECTIVE TRANSFORMATION MARKER" YEAST, CHICHESTER, SUSSEX, GB, Bd. 14, August 1998 (1998-08), Seiten 1017-1025, XP000985335 ISSN: 0749-503X

### SEQUENZPROTOKOLL

<110> Rhein Biotech Gesellschaft für neue biotechnologische Prozesse und Produkte mbH
<110> Institut für Pflanzengenetik und Kulturpflanzenforschung (IPK)
<120> Verfahren zum Herstellen von Proteinen in einer Hefe der Gattung *Arxula* und dafür geeignete Promotoren
<130> PCT1376-01966
<140>
   <141>
<160> 23
<170> PatentIn Ver. 2.1
<210> 1
   <211> 243
   <212> DNA
   <213> Arxula adeninivorans
<400> 1
   gcaatattgt gcaaagatac aagcgcattt atctatagtt ggccagcacc aaagatcatg 60
   cctttacttc tggagtctat ctaggtcgtt atcgattgtt gcatatatgc ttaaaggatt 120
   gtcgaaaaca gccccgtcga ggtttcgaag ggggacactg gcgcggcttg ccagcgacgc 180
   gtattgaaag ttggacattt aatagtttcg gagaacagag taattaactc ttcatttggc 240
   gca                                                               243
<210> 2
   <211> 724
   <212> DNA
   <213> Arxula adeninivorans
<400> 2
   gaattcatat cccgggccgt cacattagca aatccaaaaa aggcttgggt accgagcata 60
   gggcttccag tctgattaga ggatgccacc tcgagacgca ggtctgctcc atccaacgga 120
   gcgccattat cagcagacgg ttcaccgcat acatttaagg taacaaacaa tgtctggttt 180
   tgatggctcg gaattcctga tagttcgtat ctatgagttt ctcctgcctg caacgtaaac 240
   cgaatcggag tcgcctgttg tataggggta gttgtcgccg aagtgtgaaa tccttagctt 300
   tagttggaag caaaaggtcc acttcgatgc ctttatcatt gctcaatgag ctttcggatt 360
   ctttcctagg ttcccgcttc caaaagttaa ctgactggag attttccaca aattcaaacc 420
   ctgcaacgag aaaagggtca aggtcaaatg ccagtgctag gctcggccac accagcacca 480
   gcaatattgt gcaaagatac aagcgcattt atctatagtt ggccagcacc aaagatcatg 540
   ccttgtactt ctggagtcta tctaggtcgt tatcgattgt tgcatatatg cttaaaggat 600
   tgtcgaaaac agccccgtcg aggtttcgaa gggggacact ggcgcggctt gccagcgacg 660
   cgtattgaaa gttggacatt taatagtttc ggagaacaga gtaattaact cttcatttgg 720
   cgca                                                              724
<210> 3
   <211> 635
   <212> DNA
   <213> Arxula adeninivorans
<400> 3
   cacagaccag acggaatcat cctaacatta gcaattccaa atccgccgac ccgttcgtta 60
   ctcaggttga tcataagggg tcctcattcc ccaagaggag aaaacggtgt cttaattgcg 120
   cacctcttgt ttttgtagca tttttcaact aactagaccg ttcgttctat acctaattac 180
   aacatccgcc cacctttctc cttattgatt tgcaattact atccgaatag cgccgatatt 240
   gtttggtgcc gtagatgcaa tgttattacg gcaggaaaaa tatgtacgga taattgttcc 300
   gcgaaaatcc ggaatcgtaa attgtattat cggatctgat gaatcagcta tgaaagataa 360
   tatatggtat agctacgact atagtaaacc atagtcaaaa tcaaaagttt caacgttttc 420
   actgttatct catttatccc cggattggcc aagaaactaa ggttttttgg accctggaac 480
   atgcataatg cggagatctc actcggcccg acttggaagt gtaagggagg ggatccaact 540
   cggcccgact ggaaggtgca atgagtgaat ggaagtagac tggaggacta tataaacact 600
   cgcagcctgg ttacaattga cgtggacgaa acaga                            635
<210> 4
   <211> 468
   <212> DNA
   <213> Arxula adeninivorans
<400> 4
   aactctagtg ccaaatagac tgtacatatt accaaatact gatatccatt catctttcta 60
   tctgcttatc tcgagatgta cgcaatccct atctctttat ctcctaggac cttgatatca 120
   ttggcggaac gtaccgaagt tcggttggga atttccgaga cggcgcacag taactctatc 180
   tgaaccaggg acaaagatta attgaaagaa atgtctttcg gctaaaaaag agtaaggaaa 240
   gtgagaaagc aggcagcaaa gctttgcctg ggccatcggc agagcctata tttcggacat 300
   gtacgactgg ggctttcaat tagtgtctat tctaactaat tttcatgtat ttgcccgtcg 360
   gatagccgcc gggtccaccc gaccccacac ggcgaacatc cagtcaaacg gttgatataa 420
   aaagggtcta acttccgcag gtgcaggttt tttcaggtca attgtaca             468
<210> 5
   <211> 617
   <212> DNA
   <213> Arxula adeninivorans
<400> 5
   tcctgttcac tattcatctc cagacatatc tggtgtacct cgttgttgaa tttctctctt 60
   gccgtattta ccattttgaa atctcgcagc aatttgagga gtagcaacat gccttaatag 120
   gtagacctcc aagctgctgg acattatggt cataggatat tcagtccatg tagtggcatc 180
   aagatcatga aataatgaat cttaccactc tatctgtgta gcaaaggtgg cccttaaatt 240
   tctcatacag tctgatcaac tgtgatggtg ctgtacgacg cgcaaaaaaa gtaggtagaa 300
   gtatcaagtc aatatttcgg gcaattaaga ttgctcggca tttggtcctg tatattgttc 360
   tccgttatga cggtaacgcc tcatttatat cagatgggac ctcatgagga cctgtgagac 420
   ccaattatcg attatttcta ttccagttcg gctctcgact gtcgctactt atttggagcc 480
   tgttcgctga ggcgataatt taagggccaa cctgtacgac ccatttaggc aataattaag 540
   cttccagcta ttaatataaa tgcctcagac gaccctttac ccttttgtct ataatcaact 600
   ctggtcctgc aagtgag                                                617
<210> 6
   <211> 680
   <212> DNA
   <213> Arxula adeninivorans
<400> 6
   gggtcctcta gaaggaaagc atcttgatag agtattataa tggaggagcg agcgacacct 60
   actgcaagag cgctgccatg ttgataaata gcagtatcgc cgggcagctt tcttatatat 120
   ggagactttg gacatctccg ataccagtaa ttgactttat acatcttcgg aaacgaatag 180
   ccgaccatgc ttgccgaagg tattccacct gcaaggtttt ggtacaaagt atctttttgg 240
   agttgcgacg gtcttaaaat gcacgctagc atgtatccat tttcggctac tcgctttacc 300
   tttttgaaat ccacttctaa gcctgtcatt cgaggagaat ggctgctagt atgtacatta 360
   ttgatcgtgg ctgttttcaa agcagaggtc gtcaatagcc ttgtcctttg tacaccgttg 420
   atcgcatacc tcagttacct cttaatttca ccattctaac ggaatccaac ttgtgcacat 480
   aatattcatt aacagtgcca cattaacatc agttcaacgc tatctatttt ccccagctct 540
   tacagcctct tttatcatac ctttggtgtg gttagtaacg tgacaaaatt cgacgtgccg 600
   tgcacatggc atatatcacc tgataacttc cacttaccaa tgcccaaaaa ctcacagttc 660
   acccacaaaa cgccaccact                                             680
<210> 7
   <211> 629
   <212> DNA
   <213> Arxula adeninivorans
<400> 7
   ccttcgtacg gtcggtcttc atacggtttg gtcacttcag cagacactct tacaataacc 60
   ttagtcttgc ctaagctcac ctctacatgt cctaacgact ctccaaatgt aatttccaca 120
   tcgcgaaatt gcagtagctt gcgtccatca acacgggttt cctgcttcaa caggtccagg 180
   acaaagtcct tttcattatt tgacagttcg accttcatgg tatcgagtta gcatactgag 240
   cgagcactga aaagtcaatt tcgaatcacg tgagatatac gtgatcatct catctgccac 300
   tcagctgaat atatcacgtg ttatacctcg tttacaacgt ataaaaacac gacaaaagcg 360
   agactcaatt agcagtaatt gggtgattcg acagcaatga cagcgctttc gtgatcattc 420
   aattcatttc aaagtacaat cgctaccggt ctcaacttct gtcatcttta cgactcccat 480
   ccttgatttc atctctggtc atcgcgaaaa caacgtgttg aggaaaaaaa gccccaccaa 540
   ccgcggcgct cgaggcgaac ctcctctcga agacacacac atatatatgg accccctgtg 600
   agtttgaaac ttttacactc aactttaaa                                   629
<210> 8
   <211> 515
   <212> DNA
   <213> Arxula adeninivorans
<400> 8
   gagctcaact acatttccaa agaagcatta atcgaggata tcaagactga cgttcgagta 60
   gcactcaatt cgctacaacg acctgactac gaaaagtacg agaaggatga gttttggacg 120
   gataattaga cgtaagccta gactgtgtct tccccccacg tgtcggttgt tagccgggga 180
   aacccaacat ttatgacgag catagaaata gagcataatt gaatccgact gtaaagaacg 240
   aaacagaact ccacatgtcc atgtcctata cataacgcta acaacaattc tacataatcc 300
   agcgtcacta tgatccaatg ggctatggcc ccatggttaa tatattgacc cggtcccccc 360
   cttaggaatt attcaacatt agtcttaacc gtatcgggtg gctctggccc ggcccctccg 420
   gtgttatagg gttgctccgg tccgacgtgt atatatatga agcccttccc cggaaaccac 480
   tcctcgaaaa caaaatcaca tctgattaat aaaaa                            515
<210> 9
   <211> 429
   <212> DNA
   <213> Arxula adeninivorans
<400> 9
   agtatattgc gatggtgtaa tggcattgaa tgtgtgtgct ggcactggga tttgacaaag 60
   gtgtccttgg acccgtggtc ccccaccggc caactggacc cgccaactcc gtccggtata 120
   gacagaggat taaaaaataa aggaaaaagc ctctcgactc ctgaagcaac aaagttaact 180
   aaaaccacca tttttacacc gatttcggtt tgaggcaaac gagcatccgt tttacaaaaa 240
   accagcgcag ttcctagtgg aggcaagccc gagacgcgcg tttgttgtaa tcgattctga 300
   tcccggtggc agtcttgaaa agtgtgtgag tgagtgatta ttgagagtcc cgtgtttgtt 360
   gttgttggtc atcagtcccc ctttacactt tcatcgccct tttattccta ataacttgaa 420
   taacgtgtc                                                         429
<210> 10
   <211> 600
   <212> DNA
   <213> Arxula adeninivorans
<400> 10
   aagcttggca cgaaactttg caccgtcccc tacctacctc gtccctgccg ggacactatg 60
   caattgctgc agtggcctgg gattggctaa ccttccgggg cgggtagtga cgcccgcaca 120
   gctctgcacg gggattgatc ggtaactgat cttcatctga taatttcaca ccacttcatt 180
   acccaattgg gacgtgcacc ggtgggaccc gaccgccata ctcacattta ccttcggccg 240
   actggaaaaa gaaagctcgc agccgggtaa gggaacattt tatcagattg tccataaaat 300
   tgccggcaac cttccaacag cataaccttt cccgagtact tatttggcgt ccaaggtcct 360
   ttgttatatc gacatcatct tggaaccgag tttacaactg gggtgcttgc ttgactttaa 420
   agtttgatcg ctcacgatcg ttgataagca aagtcggtta aatttcacct agcattcatc 480
   gagggggttc tttgtggttg attttatatt ctttcccgtc ccccgtacag taattttgct 540
   catttgagtt gttttttctg acaaagtgct tgagcatatt taccttgtgt gcataatatt 600
<210> 11
   <211> 1009
   <212> DNA
   <213> Arxula adeninivorans
<400> 11
   aatattatgc acacaaggta aatatgctca agcactttgt cagaaaaaac aactcaaatg 60
   agcaaaatta ctgtacgggg gacgggaaag aatataaaat caaccacaaa gaaccccctc 120
   gatgaatgct aggtgaaatt taaccgactt tgcttatcaa cgatcgtgag cgatcaaact 180
   ttaaagtcaa gcaagcaccc cagttgtaaa ctcggttcca agatgatgtc gatataacaa 240
   aggaccttgg acgccaaata agtactcggg aaaggttatg ctgttggaag gttgccggca 300
   attttatgga caatctgata aaatgttccc ttacccggct gcgagctttc tttttccagt 360
   cggccgaagg taaatgtgag tatggcggtc gggtcccacc ggtgcacgtc ccaattgggt 420
   aatgaagtgg tgtgaaatta tcagatgaag atcagttacc gatcaatccc cgtgcagagc 480
   tgtgcgggcg tcactacccg ccccggaagg ttagccaatc ccaggccact gcagcaattg 540
   catagtgtcc cggcagggac gaggtaggta ggggacggtg caaagtttcg tgccaagctt 600
   tgtgagcgcc aagagagcct acagatgtcg agaccacaga ttttattccc gaggatttcc 660
   gactaaagat cacatctgat tatgccgttt tgggaaggtg ccgtttgcgg taaaatctga 720
   tcaaccccta cacggcagtt ctgaaagctg agtggttgag aggtgcaggt aagggagacg 780
   gcaattgggt ctagaaccca acaaaaaagt aactcggagc caatctaatc tgatcaagga 840
   gaaaaagtaa aaataaacca aagcaaaaaa gtaaccggct gccgaatccc tttttcgggt 900
   gcgtccactg atgttggggc ccgaaacggg gatcgtcctg gtgctgaaat ataaagcaat 960
   tagcaatccc accccttaag gggacatcta gacactccag cgcacatca          1009
<210> 12
   <211> 507
   <212> DNA
   <213> Arxula adeninivorans
<400> 12
   cggtatgccc tggaagggta atgctgctgc caacgatgat gacttcctcg acctcactgg 60
   ccagaacgcc caggcccctg acttgcctgc tggtttcact gcccgaggta tcgtggctct 120
   ggtcttctcc tgtatttctg cattcctggg tatgggaatg atcacctggt acggactcag 180
   cgacatttcc aacactgagc gaaaggtgct agagcgagaa ggtgtcattt ccgagggaga 240
   gcaggtcgac cctattgacc ccagcaccgt cgctcaccct catcaccacg aaaattagaa 300
   gagttaattg tcacgattgt tttgtttgta gttaggaaca tgaactggtc tccttatcat 360
   aataatttcg cactaaatct ttggaatatc ttatcttatc gggtacggct cgtccgagga 420
   gtaccgaggt aggtgactga aaatttcacg ttttgaaagg tccccaaggc agtctttcac 480
   gaaccgtgtc tcccccttga caacact 507
<210> 13
   <211> 787
   <212> DNA
   <213> Arxula adeninivorans
<400> 13
   gtaatctctg aaaattccca acgagaagat ggtgaaagca agcaagtagc atcccaactt 60
   agggctaccg gcaatcttag tcagtacctt ggtcttgtac tctgtactgt tagtcttgtc 120
   caataactaa tgatcatcga atccttctta tagcatacct agtctggcga caatgttcca 180
   gaagattgga ttgaaggcga ccgaagccaa cacccctggg ttagcaactt gagtcaaagt 240
   aactccacgt cgtatacgta cacttgagag agtcctgagt gaggtcaatt cgagcaattt 300
   ctgcaacctt tgctaccgag tccattataa ttcagtaatc ctgtaatact aaaggttcac 360
   ttggcaacct gattttccag gggacgttgc ttgaatgtga ccccgcggct ttattggctc 420
   tagggaaaat tccacggttt ctatatactt cttgccgcca tagttcatca ttctagaagc 480
   tcgattagca tgacacacta gaatagcaca gtattctgtc ctactccctc tgatcaattc 540
   catcattgtt aaaggcgttt gagaatttgg tagactcagt aacaatgccc tcacttcctg 600
   cgcgcgccgc agaagtgatc gcctatccaa acttcctaat taagacatac aatacacacg 660
   caggatcaaa gtgtccaaca ggaccagaag gaataatgcc aaccaattgc agcaattgaa 720
   gcgaggtggg gatgaaatga ggtatatata gcaaataaat atccgtttgt attgcacata 780
   tcagcta 787
<210> 14
   <211> 688
   <212> DNA
   <213> Arxula adeninivorans
<400> 14
   cggcaaactt gtacctcacg cccaaaacgg aaaagagtgt ttcggatacg ggccgccatg 60
   atgactccgg tccggaggtt gacagctcag gggcaccaga tagaccttat tctgaagaaa 120
   ccagcacttg tagccagtaa cggtcattgc agactgccat agacataggt tatttgtaat 180
   tttccgtctt tacaatgccc attgagcaag cttttctcat ccaatccaga atgatatacg 240
   gtaactttcg ccataatagt accggcgatc tcccagagcc gcacatgagc ctcttggaca 300
   ttcggcacac cgatcatcca cctcctctct tattgccttg ccatgggcgt tcgatgagta 360
   accctcttga agtgggtaca attcacgcct gtttgccact ccgtagacga caatagaaga 420
   tcacactatc cagctgtact tgacatacac gttcaccggg tcctaaacta ctgcctggtt 480
   ctccctcttt tctttggacg ttttcctcaa cgggctcgcc tacctcgcat acgatgcatg 540
   cgcctcacaa tgtaaatact tgtaaatacg aaaaagaaat tattgggctt cgtccccgcc 600
   gatcatcgtt gtgatcatgg aatcaaaagt atataaactg ccaaggatcc cccaagctta 660
   aaggtttcat ccgcgagtta gtgctgaa 688
<210> 15
   <211> 2175
   <212> DNA
   <213> Arxula adeninivorans
<400> 15
   ttttcaatcg acgaattgca attgacaaag agagaacatg ctcttaagac tgaacatctt 60
   atcagacaag ggccgcatcg agcggctacc tcgtaattgt ggtcagtcat tgacaaccat 120
   tagcaagcca ttggcaacgg gacaacgcag gcgaatctca ctcgcagtta ctgcctgcaa 180
   tgacaattgc tcctcttcgc gcaaacataa tagactttga taagcaccac aatcgcttct 240
   tccctcggtc gtactggcaa atttcatgca tgaatgcggc tgcagcctcc tgtcgatatg 300
   ggacggagct ctgttgcttt cggggccctt tccaagcttc aatccactaa tcggagaatg 360
   ttcatcaact ggctcagcta atcagatagg tcaacttgtt ccccagcggt ttcaccacat 420
   gctgtccttt cctccccccg ctcggaagga tacgcagaat gctttatcgt ggggctgccc 480
   cgggccgaaa ccggcatttt gggttgactc ttttaaggaa cgacatgccc aggggactct 540
   ccactcacaa accctttcaa aaaccatgag caaaaacatt atcattctgt ctggagatca 600
   cgtaggacct gaggtcactg ctgaggccat taaggtgctc gaagccatta cccaggctcg 660
   tcctaacgta aagttcaact tcgatcacaa gctaattgga ggagctgcta tcgatgccac 720
   cggatctccc cttcctgatg agactctgga ggcttctaag aaagccgatg ccgtgttgct 780
   gggagctgta ggaggtccca agtggggaac tggtgccgtc cgtcctgagc agggtctgct 840
   caagattcga aaggaactca acctgtatgc caacctgcgt ccttgcaatt tcatgtcaga 900
   aaagcttctg gatctgtccc ctctcaggtc cgagattgtc aagggaacca acttcactgt 960
   tgttcgagag ctcgtgggag gcatctattt tggtacccga aaggaagatg agggcaatgg 1020
   agaggcatgg gacactgaaa aatacactgt ggaggaggta aagcgaatta ctcgaatggc 1080
   tgcttttctg gcccttcagt ctaaccctcc tttgcctgtg tggagtttgg acaaggctaa 1140
   cgtattggct tcttctcgtc tgtggagaaa gactgtgacc gagacaattg agaaggagtt 1200
   cccacagctg accctcaacc accagctgat tgattctgcg gccatgattc ttatccagaa 1260
   cccatccaag atgaatggag taattgtcac ttcgaacatg tttggagaca tcatttctga 1320
   tgaggcctca gttattcctg gatctctggg tctgctgcct tctgcctctc tgtcctctct 1380
   tcccgacaag aacacggcct ttggactgta cgagccttgt cacggaagtg cccccgactt 1440
   gcctcctaat aaggtgaacc ccattgccac aattttgtct gctgccatga tgttgcgact 1500
   ttctttgaac ctcaaggagg aagctgatgc tgttgagaag gccgtcagta aggtaattga 1560
   caatggcatt gtcaccgccg atctcaaggg tgcttcatct actaccgagg ttggagatgc 1620
   cgttgctgct gaggtacaga agcttcttaa ataaattcat gaatagtatt aatgattggc 1680
   aataaattgc aaaagtaacc aaataaagca aaaaaaatgg taataaattg caaaaaatag 1740
   gcgaagtata taggtatata taaaaaacac ccacgctggg ggtcgaaccc agaatcttct 1800
   gattagaagt cagacgcgtt gccattacgc cacgcaggca aagcgataaa aaactctagg 1860
   ttttaggctt acaaatttgg attagaaaaa tcaagccaaa atcggcccga tttgggcatt 1920
   aacacaaaaa ggactgtcta ataaacagat tttaagtcta agtctaaggc gttgacactg 1980
   attttaactg aaactgggcg ttaaaactgg aagccattca cgctattatt tgtaaatgta 2040
   ctgtccgagc caatcttcgc aaaaattgat agaattagtc gttattagat gccaatggcc 2100
   tatttggtaa cgcctggaga tagccaggag cgctaagggg tttcctcaca attaataggg 2160
   actgtatcta gacac                                                  2175
<210> 16
   <211> 2175
   <212> DNA
   <213> Arxula adeninivorans
<400> 16
   ttttcaatcg acgaattgca attgacaaag agagaacatg ctcttaagac tgaacatctt 60
   atcagacaag ggccgcatcg agcggctacc tcgtaattgt ggtcagtcat tgacaaccat 120
   tagcaagcca ttggcaacgg gacaacgcag gcgaatctca ctcgcagtta ctgcctgcaa 180
   tgacaattgc tcctcttcgc gcaaacataa tagactttga taagcaccac aatcgcttct 240
   tccctcggtc gtactggcaa atttcatgca tgaatgcggc tgcagcctcc tgtcgatatg 300
   ggacggagct ctgttgcttt cggcgccctt tccaagcttc aatccactaa tcggagaatg 360
   ttcatcaact ggctcagcta atcagatagg tcaacttgtt ccccagcggt ttcaccacat 420
   gctgtccttt cctccccccg ctcggaagga tacgcagaat gctttatcgt ggggctgccc 480
   cgggccgaaa ccggcatttt gggttgactc ttttaaggaa cgacatgccc aggggactct 540
   ccactcacaa accctttcaa aaaccatgag caaaaacatt atcattctgt ctggagatca 600
   cgtaggacct gaggtcactg ctgaggccat taaggtgctc gaagccatta cccaggctcg 660
   tcctaacgta aagttcaact tcgatcacaa gctaattgga ggagctgcta tcgatgccac 720
   cggatctccc cttcctgatg agactctgga ggcttctaag aaagccgatg ccgtgttgct 780
   gggagctgta ggaggtccca agtggggaac tggtgccgtc cgtcctgagc agggtctgct 840
   caagattcga aaggaactca acctgtatgc caacctgcgt ccttgcaatt tcatgtcaga 900
   aaagcttctg gatctgtccc ctctcaggtc cgagattgtc aagggaacca acttcactgt 960
   tgttcgagag ctcgtgggag gcatctattt tggtacccga aaggaagatg agggcaatgg 1020
   agaggcatgg gacactgaaa aatacactgt ggaggaggta aagcgaatta ctcgaatggc 1080
   tgcttttctg gcccttcagt ctaaccctcc tttgcctgtg tggagtttgg acaaggctaa 1140
   cgtattggct tcttctcgtc tgtggagaaa gactgtgacc gagacaattg agaaggagtt 1200
   cccacagctg accctcaacc accagctgat tgattctgcg gccatgattc ttatccagaa 1260
   cccatccaag atgaatggag taattgtcac ttcgaacatg tttggagaca tcatttctga 1320
   tgaggcctca gttattcctg gatctctggg tctgctgcct tctgcctctc tgtcctctct 1380
   tcccgacaag aacacggcct ttggactgta cgagccttgt cacggaagtg cccccgactt 1440
   gcctcctaat aaggtgaacc ccattgccac aattttgtct gctgccatga tgttgcgact 1500
   ttctttgaac ctcaaggagg aagctgatgc tgttgagaag gccgtcagta aggtaattga 1560
   caatggcatt gtcaccgccg atctcaaggg tgcttcatct actaccgagg ttggagatgc 1620
   cgttgctgct gaggtacaga agcttcttaa ataaattcat gaatagtatt aatgattggc 1680
   aataaattgc aaaagtaacc aaataaagca aaaaaaatgg taataaattg caaaaaatag 1740
   gcgaagtata taggtatata taaaaaacac ccacgctggg ggtcgaaccc agaatcttct 1800
   gattagaagt cagacgcgtt gccattacgc cacgcaggca aagcgataaa aaactctagg 1860
   ttttaggctt acaaatttgg attagaaaaa tcaagccaaa atcggcccga tttgggcatt 1920
   aacacaaaaa ggactgtcta ataaacagat tttaagtcta agtctaaggc gttgacactg 1980
   attttaactg aaactgggcg ttaaaactgg aagccattca cgctattatt tgtaaatgta 2040
   ctgtccgagc caatcttcgc aaaaattgat agaattagtc gttattagat gccaatggcc 2100
   tatttggtaa cgcctggaga tagccaggag cgctaagggg tttcctcaca attaataggg 2160
   actgtatcta gacac                                                  2175
<210> 17
   <211> 536
   <212> DNA
   <213> Arxula adeninivorans
<400> 17
   atcttctgat ccgtgtgtcg ctttactttt attctctctg tcgagcatta ctctagttgg 60
   tacgactggt atgactggta tttgtggatc ataactcaca ttagcacagg cacgtgacac 120
   tggaaccgcg ataaaatgat actggtaaat ggacaccaag ctatcaaccc aatacataag 180
   actcataaaa agtagcacac atacacacac acacacccac acaacagcaa tacatgtact 240
   caacacctca cctcgcctac cgttcaccag gttgttggac tgggattgca ccgagccgca 300
   cgcctgacgg caccacacgg ctttaaaccc tcgctgcagg ttccaccatg gcaaaatggc 360
   tcttgtccca aagcacgcgc cacacaattg ggagggaaaa caacaccaca attggggccc 420
   agttttggag caaaatatgt ggccaatcac ggcgctgtgc cgcgccaaac ctatataaac 480
   cctttgccac cctccttgtg tttttttttt cttcttgata ataacataaa cacatc     536
<210> 18
   <211> 281
   <212> DNA
   <213> Arxula adeninivorans
<400> 18
   ttaacttata ctaacaatca attgactatg ttacaaaaat ctttcctgac cgttaacggc 60
   ctgtctcacc ttgctcagta tttatccgag gatggttcga ctgcctaatg gggtaaagcc 120
   ggagcacggc tagcatgtca tcggtatcgc ccttttgggt actaactctg tggggtaaaa 180
   agcggacttg attgttccgg ttggattgca aaatatttaa gcattgagtt tccataaaga 240
   tgtgaagatt tattcaattt atccattacc ataatatagc a                     281
<210> 19
   <211> 257
   <212> DNA
   <213> Arxula adeninivorans
<400> 19
   cctattgacc ccagcaccgt cgctcaccct catcaccacg aaaattagaa gagttaattg 60
   tcacgattgt tttgtttgta gttaggaaca tgaactggtc tccttatcat aataatttcg 120
   cactaaatct ttggaatatc ttatcttatc gggtacggct cgtccgagga gtaccgaggt 180
   aggtgactga aaatttcacg ttttgaaagg tccccaaggc agtctttcac gaaccgtgtc 240
   tcccccttga caacact                                               257
<210> 20
   <211> 1164
   <212> DNA
   <213> Arxula adeninivorans
<400> 20
   tttcgcgtcc tggcggttgt tttgtggtat gagcgttgat gtatttgatc ggtaatcgcg 60
   tttaatcttc tgatccgtgt gtcgctttac ttttattctc tctgtcgagc attactctag 120
   ttggtacgac tggtatgact ggtatttgtg gatcataact cacattagca caggcacgtg 180
   acactggaac cgcgataaaa tgatactggt aaatggacac caagctatca acccaataca 240
   taagactcat aaaaagtagc acacatacac acacacacac ccacacaaca gcaatacatg 300
   tactcaacac ctcacctcgc ctaccgttca ccaggttgtt ggactgggat tgcaccgagc 360
   cgcacgcctg acggcaccac acggctttaa accctcgctg caggttccac catggcaaaa 420
   tggctcttgt cccaaagcac gcgccacaca attgggaggg aaaacaacac cacaattggg 480
   gcccagtttt ggagcaaaat atgtggccaa tcacggcgct gtgccgcgcc aaacctatat 540
   aaaccctttg ccaccctcct tgtgtttttt ttttcttctt gataataaca taaacacatc 600
   atgtctgggt aggtaaaaaa actgggtttg ataatttttt tgctgcagta ctaactaaag 660
   acgcggaaaa ggaggaaaag gacttggaaa gggaggcgct aagcgtcacc gaaagattct 720
   acgtgacaac attcagggta ttaccaagcc cgccatccgt cgtcttgctc gacgaggagg 780
   tgtcaagcgt atctcggccc tcatttatga ggagacccga tcggtgctca agaccttttt 840
   ggagtctgtg atccgagatg ccgtcaccta cactgagcac gccaagagaa agactgtgac 900
   ttctcttgat gtcgtgtacg ctctcaagcg acagggacga actctttatg gttttggagg 960
   ttaatttttt tatttaagga taatttatgg gtttaacact acaacactta gcatacacta 1020
   gagcactgaa cgtatccttg aactcgtaac ctatactaga acacgtagac atatatttgc 1080
   cacttgccac ctccgtgacc cctggactgc cggttccatt tgaccgtagc cgccactgca 1140
   atgtcgtggt agcatttggt gatg 1164
<210> 21
   <211> 103
   <212> PRT
   <213> Arxula adeninivorans
<400> 21
   Met Ser Gly Arg Gly Lys Gly Gly Lys Gly Leu Gly Lys Gly Gly Ala
   1               5                   10                  15
   Lys Arg His Arg Lys Ile Leu Arg Asp Asn Ile Gln Gly Ile Thr Lys
               20                  25                  30
   Pro Ala Ile Arg Arg Leu Ala Arg Arg Gly Gly Val Lys Arg Ile Ser
           35                  40                  45
   Ala Leu Ile Tyr Glu Glu Thr Arg Ser Val Leu Lys Thr Phe Leu Glu
       50                  55                  60
   Ser Val Ile Arg Asp Ala Val Thr Tyr Thr Glu His Ala Lys Arg Lys
   65                  70                  75                  80
   Thr Val Thr Ser Leu Asp Val Val Tyr Ala Leu Lys Arg Gln Gly Arg
                   85                  90                  95
   Thr Leu Tyr Gly Phe Gly Gly
               100
<210> 22
   <211> 1811
   <212> DNA
   <213> Arxula adeninivorans
<400> 22
   ctgtgagtgg atctgcgtag acgataagat gaatgtttgg gaaaccagag aacaggggcg 60
   tcccaatgtc gaagaaatga caataagttt cgctggtaat gggccatgct gggttgcagc 120
   gtgcggacca gatccattgc tacgggcggt gcaaaatggt gtggcaaagc tcatctccga 180
   tggccacgac gtgcatcttc atattgaggc ctttggctgg taattaactt atactaacaa 240
   tcaattgact atgttacaaa aatctttcct gaccgttaac ggcctgtctc accttgctca 300
   gtatttatcc gaggatggtt cgactgccta atggggtaaa gccggagcac ggctagcatg 360
   tcatcggtat cgcccttttg ggtactaact ctgtggggta aaaagcggac ttgattgttc 420
   cggttggatt gcaaaatatt taagcattga gtttccataa agatgtgaag atttattcaa 480
   tttatccatt accataatat agcaatggct gcacaagtcg aggaacaggt gcttaatctc 540
   cgagctcaag cggatcacaa tccctcgttt gttctcaaaa agcccttgga attggggttt 600
   gaagagcgcc cagtgcctgt gattactgac cctcgagatg ttaaaatcca ggtcaagaag 660
   acgggaatct gcggatctga tgttcacttt tggcaacatg gtcgtattgg tgactacgta 720
   gtggaaaagc caatggtcct aggccatgag tccagtggag tggtggttga agttggttct 780
   gaggtcacct ccctcaaggt gggagaccga gtagctatgg aacctggagt gccggacagg 840
   cgctcaaagg aatataagat gggacgatat catttgtgcc ctcatgtgcg ctttgcagcc 900
   tgccctccca ctgacggaac gctctgcaag tattacacac ttcccgagga cttctgcgtt 960
   aaactgcctg aaaacgtaga ttttgaggaa ggagcattgg tggagccatt gagtgtagca 1020
   gtccacaccg ctaggcttct tggtatctac cctggctcca aggtggtcgt gttcggagca 1080
   ggaccaattg gccagctgtg cattggagtg tgcaaggcat ttggtgccag tataattggc 1140
   gcggtggatt tgtttgagca aaagctagag accgcaaagg agtttggtgc atctcacacg 1200
   tatgtccctc agaaagggga ctcacatgac gagactgccc acaaaattct cgagctcctg 1260
   cccaacaagc aggccccaga tgtggtcatt gatgccagtg gagctgagca atctatcaac 1320
   gcaggaattg agcttctaga gagaggtgga acctttggac aggtggccat gggccgaact 1380
   gactacattc agtttgccgt ttctcgtatg gccatgaagg agattcgatt ccagggagtc 1440
   ttccgatata cctatggaga ttatgagctc gcaacccagt tgattggtga tggaaagatc 1500
   cctgtaaaga aattggtcac ccataggcgg ccatttgaaa aggccgagga ggcatatgaa 1560
   ttggtcaaga gcggtgttgc agtatataat tgatgcctac gattcattgt tttcggacac 1620
   agttagttgt tttccttttc ccatctattc acattgttga actgctattc ataataacac 1680
   tatattggtc gtagtcccgt actgttgaat acaggatctc ttcattccct cagcgggcat 1740
   ttaagggcac tttgatatgg ccttaggttg ggcttcttgg gctctctcgc gatctcgcct 1800
   agcgaagatc t                                                    1811
<210> 23
   <211> 368
   <212> PRT
   <213> Arxula adeninivorans
<400> 23
   Met Ala Ala Gln Val Glu Glu Gln Val Leu Asn Leu Arg Ala Gln Ala
   1 5 10 15
   Asp His Asn Pro Ser Phe Val Leu Lys Lys Pro Leu Glu Leu Gly Phe
   20 25 30
   Glu Glu Arg Pro Val Pro Val Ile Thr Asp Pro Arg Asp Val Lys Ile
   35 40 45
   Gln Val Lys Lys Thr Gly Ile Cys Gly Ser Asp Val His Phe Trp Gln
   50 55 60
   His Gly Arg Ile Gly Asp Tyr Val Val Glu Lys Pro Met Val Leu Gly
   65 70 75 80
   His Glu Ser Ser Gly Val Val Val Glu Val Gly Ser Glu Val Thr Ser
   85 90 95
   Leu Lys Val Gly Asp Arg Val Ala Met Glu Pro Gly Val Pro Asp Arg
   100 105 110
   Arg Ser Lys Glu Tyr Lys Met Gly Arg Tyr His Leu Cys Pro His Val
   115 120 125
   Arg Phe Ala Ala Cys Pro Pro Thr Asp Gly Thr Leu Cys Lys Tyr Tyr
   130 135 140
   Thr Leu Pro Glu Asp Phe Cys Val Lys Leu Pro Glu Asn Val Asp Phe
   145 150 155 160
   Glu Glu Gly Ala Leu Val Glu Pro Leu Ser Val Ala Val His Thr Ala
   165 170 175
   Arg Leu Leu Gly Ile Tyr Pro Gly Ser Lys Val Val Val Phe Gly Ala
   180 185 190
   Gly Pro Ile Gly Gln Leu Cys Ile Gly Val Cys Lys Ala Phe Gly Ala
   195 200 205
   Ser Ile Ile Gly Ala Val Asp Leu Phe Glu Gln Lys Leu Glu Thr Ala
   210 215 220
   Lys Glu Phe Gly Ala Ser His Thr Tyr Val Pro Gln Lys Gly Asp Ser
   225 230 235 240
   His Asp Glu Thr Ala His Lys Ile Leu Glu Leu Leu Pro Asn Lys Gln
                   245                 250                 255
   Ala Pro Asp Val Val Ile Asp Ala Ser Gly Ala Glu Gln Ser Ile Asn
               260                 265                 270
   Ala Gly Ile Glu Leu Leu Glu Arg Gly Gly Thr Phe Gly Gln Val Ala
           275                 280                 285
   Met Gly Arg Thr Asp Tyr Ile Gln Phe Ala Val Ser Arg Met Ala Met
       290                 295                 300
   Lys Glu Ile Arg Phe Gln Gly Val Phe Arg Tyr Thr Tyr Gly Asp Tyr
   305                 310                 315                 320
   Glu Leu Ala Thr Gln Leu Ile Gly Asp Gly Lys Ile Pro Val Lys Lys
                   325                 330                 335
   Leu Val Thr His Arg Arg Pro Phe Glu Lys Ala Glu Glu Ala Tyr Glu
               340                 345                 350
   Leu Val Lys Ser Gly Val Ala Val Lys Cys Ile Ile Asp Gly Pro Glu
           355                 360                 365

## Beschreibung

Die vorliegende Erfindung betrifft ein Verfahren zum Herstellen von einem oder mehreren Proteinen in einer Wirtszelle der Hefegattung *Arxula* und ein Nukleinsäure-Molekül, das einen dafür geeigneten Promotor umfasst. Ferner betrifft die vorliegende Erfindung einen Expressionsvektor, eine Wirtszelle und einen Kit sowie die Verwendung derselben.

Hefen werden seit den 80er Jahren als Produzenten heterologer Proteine genutzt. So wurden in *S*. *cerevisiae* z.B. HbsAg, α1-Antitrypsin, Hirudin, Glucoamylase, EBV Envelope Protein, h-SOD, HSA, h-Interferon α und h-EGF als heterologe Proteine synthetisiert (Gellissen & Hollenberg 1997). Da trotz Erfolgen bei der heterologen Genexpression Gene, die vor allem sekretorische Proteine kodieren, sich in der Regel nur unbefriedigend in *S. cerevisiae* exprimieren ließen, wurde schon frühzeitig damit begonnen, Wirts-Vektor-Systeme mit nicht-konventionellen Hefen, wie *Kluyveromyces lactis, Hansenula polymorpha* und *Pichia pastoris* zu etablieren. Hiermit ließen sich meist höhere Ausbeuten an heterologen Proteinen erreichen. So gelang es z.B. Prochymosin in *K. lactis* in einer Größenordnung von einigen g/l zu produzieren. Diese Systeme erfüllen jedoch nicht alle an sie gestellten Anforderungen. So treten besonders bei der Synthese heterologer sekretorischer Proteine immer wieder Schwierigkeiten auf, die den Einsatz von Hefen zur heterologen Genexpression einschränken (Sudbery 1996, Hollenberg & Gellissen 1997, Gellissen & Hollenberg 1997).

Zur Expression heterologer Gene wurden bisher nur Hefearten verwendet, die entweder Hefezellen oder Pseudomyzel bilden. Da filamentöse Pilze, wie *Aspergillus*-Arten, als weitaus bessere "Sezemierer" insbesondere für Glycoproteine bekannt sind, wäre die Nutzung von Hefen, die auch Myzelien bilden können, eine Möglichkeit, die heterologe Genexpression zu verbessern. Leider sind die meisten myzelbildenden Hefearten entweder pathogene Arten (z.B. *Candida albicans*) oder bisher kaum untersucht. Eine nichtpathogene Hefe, die sowohl genetisch und molekularbiologisch gut untersucht als auch in der Lage ist, unter bestimmten Kultivierungsbedingungen Myzel zu bilden, ist *Arxula.*

Diese Hefegattung, mit ihrer bekanntesten Art, *A. adeninivorans,* wurde erstmals 1984 von Middelhoven et al. beschrieben. Sie bezeichneten diese dimorphe, xerotolerante, ascomycete, anamorphe, arthroconidale und Nitrat-positive Hefe zunächst als *Trichosporon adeninovorans,* bevor sie 1990 von van der Walt in *A. adeninivorans* umbenannt wurde. Diese Art zeichnet sich durch außergewöhnliche biochemische Eigenschaften aus. Hervorzuheben sind z.B. das große Spektrum von Substanzen (u.a. Adenin und andere Purine), die als C- bzw. N-Quelle genutzt werden können, die Resistenz gegenüber NaCl (Wachstum bis zu einer NaCl-Konzentration von 17,5%) und die für Hefen außerordentlich hohe Thermotoleranz. Die maximale Wachstumsgeschwindigkeit in zuckerhaltigem Medium wird bei Temperaturen zwischen 37°C und 41°C erreicht. Jedoch auch bei Temperaturen über 45°C lässt sich *A*. *adeninivorans* noch kultivieren, ohne dass eine vorherige Adaptation an diese extrem hohe Wachstumstemperatur notwendig ist.

Um Hefen der Gattung *Arxula* und insbesondere *A. adeninivorans* zur Proteinherstellung nutzen zu können, ist es notwendig, Transkriptionspromotoren zu isolierten, mit denen man eine starke Expression von heterologen Genen erzielen kann. Bisher wurden jedoch nur Promotoren genutzt, z.B. *AILV1*-Promotor, die zu einer sehr schwachen Expression von heterologen Genen (z.B. *XyIE*-Gen von *Pseudomonas putida*) in *Arxula* führen, und die sich deshalb zur Proteinproduktion in großem Maßstab nicht eignen. Es besteht also ein Bedarf nach einem Verfahren zum Herstellen von Proteinen in *Arxula* und dafür geeignete Promotoren.

Es ist deshalb Aufgabe dieser Erfindung, ein Verfahren zum Herstellen eines Proteins in einer Hefe der Gattung *Arxula* bereitzustellen, mit dem höhere Proteinausbeuten erhalten werden können.

Diese Aufgabe wird gelöst durch ein Verfahren zum Herstellen von einem oder mehreren Proteinen in einer Wirtszelle der Hefegattung *Arxula,* umfassend:
(i) Klonieren von mindestens einer Nukleinsäure, die ein heterologes Protein kodiert, in einen Expressionsvektor, der einen Promotor des konstitutiv exprimierten AHSB4-Gens , aus einer Hefe der Gattung *Arxula* enthält der die in SEQ ID No : 17 angegebene Sequenz umfasst, so dass die klonierte Nukleinsäure unter der Transkriptionskontrolle des Promotors steht;
(ii) Einführen des in (i) erhaltenen Expressionsvektors in eine zum Herstellen des heterologen Proteins geeignete Wirtszelle der Hefegattung *Arxula;*
(iii) Kultivieren der in (ii) erhaltenen Wirtszelle und
(iv) Emten des Proteins in an sich bekannter Weise.

Ein "induzierbarer Promotor" im Sinne der Erfindung steht für eine Nukleinsäuresequenz, die unter den jeweiligen Induktionsbedingungen, z.B. höherer Temperatur oder Salzkonzentration oder anaeroben Bedingungen, eine mindestens doppelte Inititationshäufigkeit der Transkription einer unter ihrer Kontrolle stehenden, für ein heterologes Protein kodierenden Nukleinsäuresequenz im Vergleich mit Nichtinduktionsbedingungen bewirkt. Die Transkriptionsinititationshäufigkeit kann anhand der durch Northern-Hybridisierung gemessenen Konzentration des jeweiligen Transkripts indirekt ermittelt werden. Ein "konstitutiver Promotor" im Sinne der Erfindung bezeichnet eine Nukleinsäuresequenz, die unter unterschiedlichen Bedingungen im Wesentlichen die gleiche Initiationshäufigkeit bewirkt.

Eine "Nukleinsäure, die ein heterologes Protein kodiert" bezeichnet im Sinne dieser Erfindung eine kodierende Nukleinsäuresequenz, die von einem anderen Gen als der zur Kontrolle ihrer Transkription eingesetzten Promotor stammt.

Im erfindungsgemäßen Verfahren umfasst der von dem *AHSB4*-Gen einer Hefe der Gattung *Arxula* stammende *AHSB4*-Promotor die in SEQ ID NO:17 angegebene Sequenz.

Promotoren, die von einem Gen der Hefe *Arxula adeninivorans* stammen, sind für die Ausführung des erfindungsgemäßen Verfahrens bevorzugt.

Der in dem erfindungsgemäßen Verfahren eingesetzte Expressionsvektor kann weiterhin mindestens ein Markergen enthalten. Vorzugsweise ist das Markergen das *ALEU2*-Gen von *Arxula adeninivorans* oder eine Variante desselben, die die Funktion des *ALEU2-*Gens beibehält (d.h. eine *leu2*-Auxotrophie komplementieren kann). Besonders bevorzugt ist ein Markergen mit der in SEQ ID NO:15 oder SEQ ID NO:16 angegebenen Sequenz. Andere Markergene, wie z.B. das *LYS2*-Gen von *S. cerevisiae* oder *A. adeninivorans,* oder das *AILV1*-Gen von *A. adeninivorans,* sind ebenfalls verwendbar.

Der Expressionsvektor kann weiterhin eine Sequenz umfassen, die die Integration des Vektors oder eines Teils desselben in das Genom der Wirtszelle ermöglicht, beispielsweise eine Sequenz aus der 25S-rDNA einer Hefe der Gattung *Arxula,* insbesondere *A. adeninivorans.* Alternativ kann der Expressionsvektor Sequenzen umfassen, die die Replikation des Vektors in der Wirtszelle ermöglichen, wie z.B. ARS-Sequenzen aus *S. cerevisiae, Schwanniomyces occidentalis, Hansenula polymorpha, Arxula adeninivorans, Yarrowia lipolytica, Picha pastoris, Kluyveromyces lactis* oder *Debaryomyces hansenii.*

Die Erfindung stellt ebenfalls Nukleinsäure-Moleküle bereit, die sich als Promotoren zur Expression heterologer Gene zum Herstellen von einem oder mehreren Proteinen in einer Wirtszelle der Hefegattung *Arxula* eignen.

In einer Ausführungsform der Erfindung umfasst das Nukleinsäure-Molekül: konstitutiven
(1) einen konstitutiven Promoter der aus folgenden Nukleinsäuren ausgewählt wird:
   (a) einer Nukleinsäure mit der in SEQ ID NO: 17 angegebenen Sequenz;
   (b) einer Nukleinsäure mit einer Sequenz, die mindestens 70% Identität mit einer der in (a), angegebenen Sequenzen aufweist;
   (c) einer Nukleinsäure, die mit dem Gegenstrang einer der in (a), angegebenen Nukleinsäuren unter stringenten Bedingungen hybridisiert;
   oder
(2) eine Nukleinsäure mit einer Sequenz, die zu der Sequenz einer der in (a) bis (c) angegebenen Nukleinsäuren komplementär ist.

Im Sinne der Erfindung bezieht sich der Ausdruck "% Identität" auf Identität auf DNA-Ebene, die gemäß bekannter Verfahren, z.B. der computergestützten Sequenzvergleiche (Altschul et al. 1990) bestimmt werden kann.

Der dem Fachmann bekannte Ausdruck "Identität" bezeichnet den Grad der Verwandtschaft zwischen zwei oder mehr DNA-Molekülen, der durch die Übereinstimmung zwischen den Sequenzen bestimmt wird. Der Prozentsatz der "Identität" ergibt sich aus dem Prozentsatz identischer Bereiche in zwei oder mehr Sequenzen unter Berücksichtigung von Lücken oder anderen Sequenzbesonderheiten.

Die Identität miteinander verwandter DNA-Moleküle kann mit Hilfe bekannter Verfahren bestimmt werden. In der Regel werden spezielle Computerprogramme mit den besonderen Anforderungen Rechnung tragenden Algorithmen eingesetzt. Bevorzugte Verfahren zur Bestimmung der Identität erzeugen zunächst die größte Übereinstimmung zwischen den untersuchten Sequenzen. Computerprogramme zur Bestimmung der Identität zwischen zwei Sequenzen umfassen, sind jedoch nicht eingeschränkt auf, das GCG-Programmpaket, einschließlich GAP (Devereux et al. 1984); Genetics Computer Group University of Wisconsin, Madison, (WI)); BLASTP, BLASTN und FASTA (Altschul et al. 1990). Das BLAST X Programm kann vom National Centre for Biotechnology Information (NCBI) und aus weiteren Quellen bezogen werden (BLAST Handbuch, Altschul S. et al., NCB NLM NIH Bethesda MD 20894; Altschul et al. 1990). Auch der bekannte Smith Waterman-Algorithmus kann zur Bestimmung von Identität verwendet werden.

Bevorzugte Parameter für den Sequenz-Vergleich umfassen die nachstehenden:

| | |
|---|---|
| Algorithmus: | Needleman und Wunsch (1970) |
| Vergleichsmatrix: | Übereinstimmung (matches) =+10, |
| | Nichtübereinstimmung (mismatch) = 0 |
| Lücken-Wert (Gap Penalty): | 15 |
| Lückenlängen-Wert: | |
| (Gap Length Penalty): | 1 |

Das GAP-Programm ist auch zur Verwendung mit den vorstehenden Parametern geeignet. Die vorstehenden Parameter sind die Standardparameter (default parameters) für Nukleinsäuresequenz-Vergleiche.

Weitere beispielhafte Algorithmen, Lücken-Öffnungs-Werte (gap opening penalties), Lückenausdehnungs-Werte (gap extension penalties), Vergleichsmatrizen einschließlich der im Programm-Handbuch, Wisconsin-Paket, Version 9, September 1997, genannten können verwendet werden. Die Auswahl wird von dem durchzuführenden Vergleich abhängen und weiterhin davon, ob der Vergleich zwischen Sequenzpaaren, wobei GAP oder Best Fit bevorzugt sind, oder zwischen einer Sequenz und einer umfangreichen Sequenz-Datenbank, wobei FASTA oder BLAST bevorzugt sind, durchgeführt wird.

Das Merkmal "Sequenz, die mit dem Gegenstrang einer Sequenz nach (a), hybridisiert" weist auf eine Sequenz hin, die unter stringenten Bedingungen mit dem Gegenstrang einer Sequenz mit den unter (a), angegebenen Merkmalen hybridisiert Beispielsweise können die Hybridisierungen bei 68°C in 2 x SSC oder nach dem Protokoll des Dioxygenin-Labelling-Kits der Firma Boehringer (Mannheim) durchgeführt werden.

Die in (b) angegebene Nukleinsäure kann auch mindestens, 80% oder 90% Identität mit einer der in (a), angegebenen Sequenzen aufweisen. Besonders bevorzugt weist sie 95% Identität mit einer dieser Sequenzen auf.

Ein weiteres von der Erfindung bereitgestelltes Nukleinsäure-Molekül umfasst
(1) einen konstitutiven Promotor, der aus folgenden Nukleinsäuren ausgewählt ist:
   (a) einer Nukleinsäure mit der in SEQ 10 NO:17 angegebenen Sequenz;
   (b) einer Nukleinsäure mit einer Sequenz, die mindestens 70% Identität mit der in (a) angegebenen Sequenz aufweist;
   (c) einer Nukleinsäure, die mit dem Gegenstrang der in (a) angegebenen Nukleinsäure unter stringenten Bedingungen hybridisiert;
   oder
(2) eine Nukleinsäure mit einer Sequenz, die zu der Sequenz einer der in (a) bis (c) angegebenen Nukleinsäuren komplementär ist.

Die in (b) angegebene Nukleinsäure kann auch mindestens, 80% oder 90% Identität mit der in (a) angegebenen Sequenz aufweisen. Besonders bevorzugt weist sie 95% Identität mit dieser Sequenz auf.

Das erfindungsgemäße Nukleinsäure-Molekül kann weiterhin mindestens eine unter der Transkriptionskontrolle des Promotors stehende Nukleinsäuresequenz für ein heterologes Gen umfassen. Ein "heterologes Gen", wie hier verwendet, bezeichnet eine kodierende Nukleinsäuresequenz, die von einem anderen Gen als der zur Kontrolle ihrer Transkription eingesetzten Promotor stammt. Die kodierende Nukleinsäuresequenz kann von einer Hefe der Gattung *Arxula* oder von einem anderen Organismus stammen. Beispiele für exprimierbare heterologe Gene sind unter anderem die Gene für Insulin, Phytase, HGH, Hirudin, Lactoferrin oder G-CSF.

Die Erfindung stellt ebenfalls einen Expressionsvektor bereit, der mindestens ein erfindungsgemäßes Nukleinsäure-Molekül umfasst. Vorzugsweise enthält der Expressionsvektor mindestens ein Markergen. In einer bevorzugten Ausführungsform enthält der erfindungsgemäße Expressionsvektor als Markergen das *ALEU2*-Gen von *Arxula adeninivorans* oder eine Variante desselben. Besonders bevorzugt weist das Markengen die in SEQ ID NO:15 oder SEQ ID NO:16 angegebene Sequenz auf. Andere Markergene, wie z.B. das LYS2-Gen von *S. cerevisiae* oder *A. adeninivorans,* oder das *AILV1*-Gen von *A. adeninivorans,* sind ebenfalls verwendbar.

Der erfindungsgemäße Expressionsvektor kann eine Sequenz umfassen, die die Integration des Vektors oder eines Teils desselben in das Genom einer Wirtszelle der Hefegattung *Arxula* ermöglicht, beispielsweise eine Sequenz aus der 25S-rDNA einer Hefe der Gattung *Arxula,* insbesondere *A. adeninivorans.* In einer anderen Ausführungsform umfasst der Expressionsvektor eine Sequenz, die die Replikation des Vektors in einer Wirtszelle der Hefegattung *Arxula* ermöglicht, wie z.B. ARS-Sequenzen aus *S. cerevisiae, Schwanniomyces occidentalis, Hansenula polymorpha, Arxula adeninivorans, Yarrowia lipolytica, Picha pastoris, Kluyveromyces lactis* oder *Debaryomyces hansenii.*

Ferner stellt die Erfindung eine Wirtszelle bereit, die ein erfindungsgemäßes Nukleinsäure-Molekül oder einen erfindungsgemäßen Expressionsvektor enthält. Vorzugsweise gehört die Wirtszelle der Hefegattung *Arxula* an. Besonders bevorzugt als Wirtszelle ist die Hefe *Arxula adeninivorans.*

Die Erfindung stellt weiterhin einen Kit bereit, der:
(a) einen erfindungsgemäßen Expressionsvektor, der zum Klonieren einer Nukleinsäure geeignet ist, welche ein heterologes Protein kodiert, und
(b) eine zur Induktion des Promotors und zum Herstellen des heterologen Proteins geeignete Wirtszelle
umfasst.

Das Nukleinsäure-Molekül, der Expressionsvektor, die Wirtszelle und der Kit gemäß der Erfindung können zur Expression eines Gens unter der Kontrolle des Promotors oder zum Herstellen von einem oder mehreren Proteinen verwendet werden.

Die folgenden Figuren und Beispiele erläutern die Erfindung.

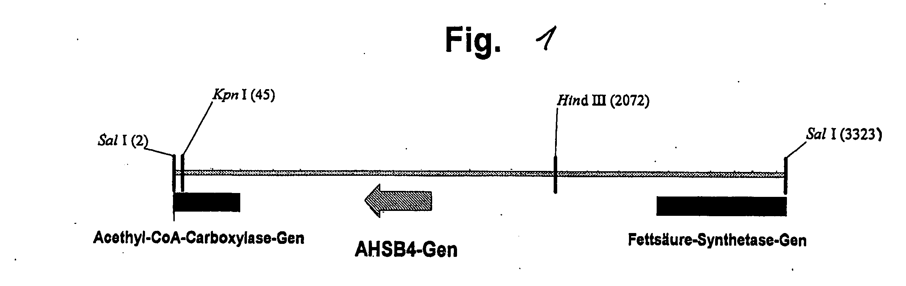

Fig. 1 zeigt die Gen- und Restriktionskarte des 3323 Bp großen DNA-Fragmentes mit dem *AHSB4*-Gen.

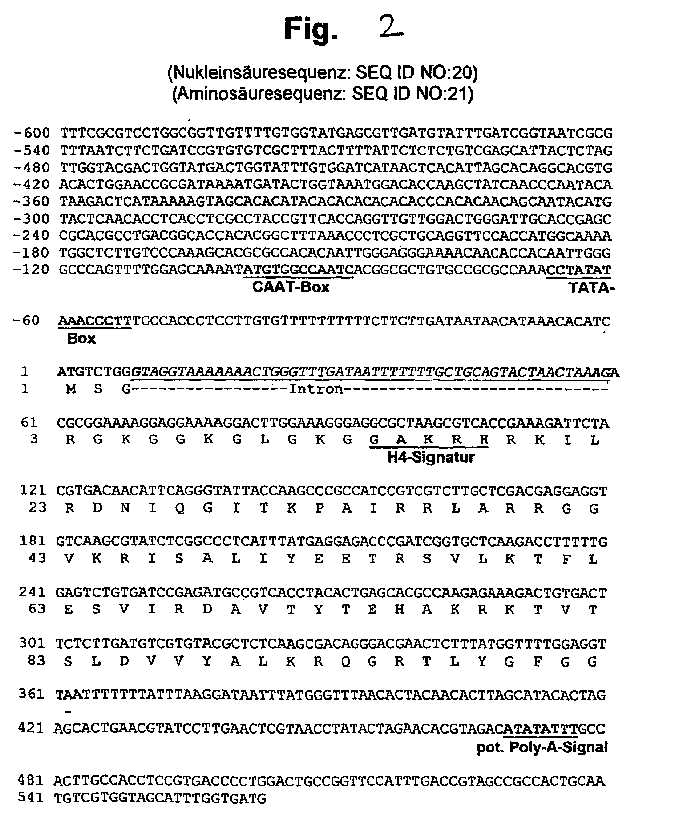

Fig. 2 zeigt die Nukleotidsequenz (einschließlich Promotor und Terminatorbereich) (SEQ ID NO:20) und die davon abgeleitete Aminosäuresequenz (SEQ ID NO:21) des *AHSB4-*Gens.
Fig. 3 zeigt die *AHSB4*-Transkriptanalyse von *A. adeninivorans* LS3. Dazu wurden die Hefezellen (A) für 20, 36, 40, 60 und 70 h (1-5) in HMM mit 2% Maltose bei 30°C kultiviert bzw. (B) die Zellen nach 45stündiger Kultivierung im HMM mit 2% Maltose in HMM mit 2% Maltose und 5% NaCl umgesetzt und für weitere 0, 0,5, 1,2, und 19 h (1-5) bei 30°C kultiviert. Anschließend wurde aus diesen Zellen die RNA isoliert und mittels Northem-Hybridisierung die *AHSB4*-Transkriptkonzentration bestimmt. Als radioaktiv markierte Sonde wurde ein 0,8 Kbp *Eco*RI/*Xho*I-DNA-Fragment mit dem *AHSB4*-Gen genutzt.
Fig. 4 zeigt die Gen- und Restriktionskarte des 2838 Bp großen DNA-Fragmentes mit dem *ALEU2*-Gen.
Fig. 5 zeigt die Gen- und Restriktionskarte des 2185 Bp großen DNA-Fragmentes mit dem *ALEU2m-*Gen.

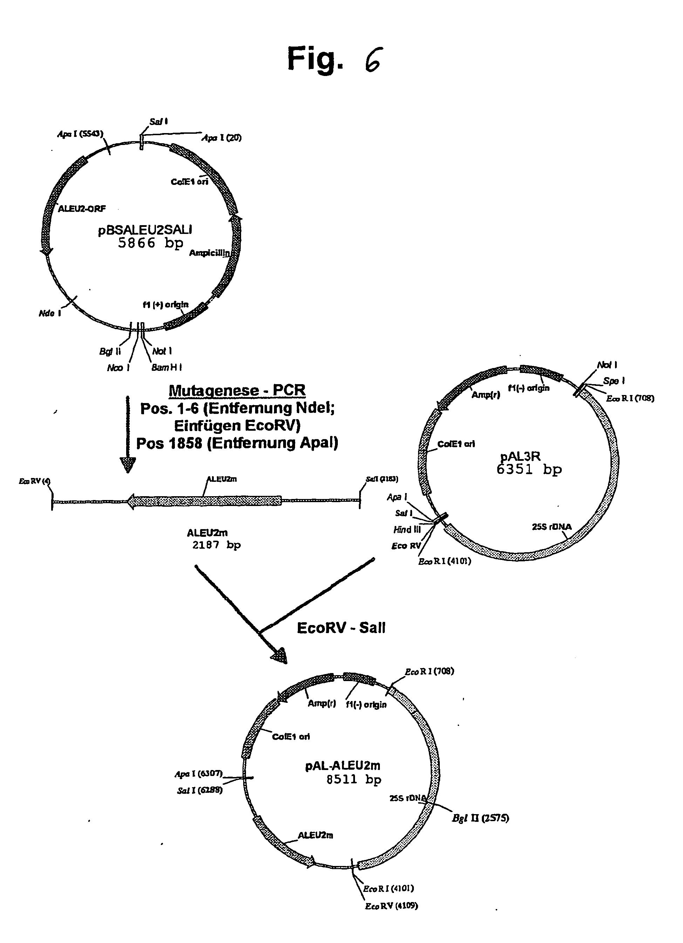

Fig. 6 zeigt das Konstruktionsschema für das Plasmid pAL-ALEU2m.

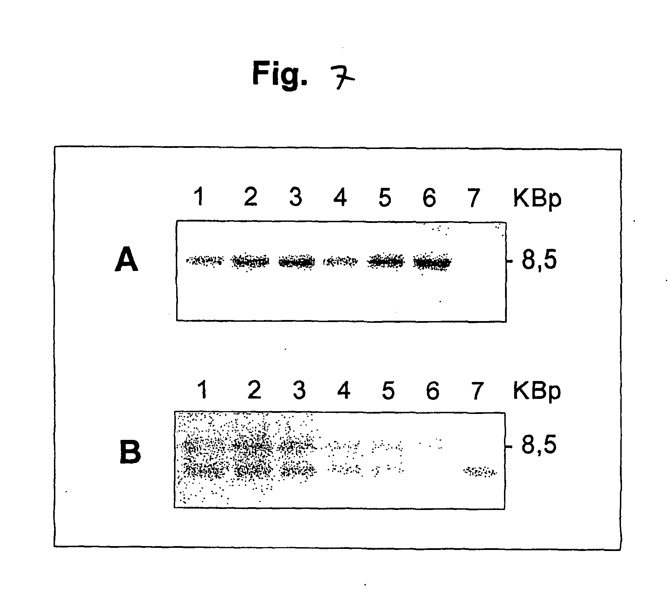

Fig. 7 zeigt das Ergebnis einer Southern-Hybridisierung zur Bestimmung der ins Genom von *A. adeninivorans* G1211/pAL-ALEU2m integrierten Plasmid-Kopiezahl. Chromosomale DNA von (1) G1211/pAL-ALEU2m-1, (2) G1211/pAL-ALEU2m-2, (3) G1211/pAL-ALEU2m-3, (4) G1211/pAL-ALEU2m-4, (5) G1211/pAL-ALEU2m-5, (6) G1211/pAL-ALEU2m-6 und G1211 (7) wurde mit *Bgl*II restringiert und gegen das radioaktiv markierte Plasmid (A) pUC19 (gespalten mit *Bam*HI) bzw. gegen ein *Bgl*II-DNA-Fragment mit dem *ALEU2m*-Gen hybridisiert.

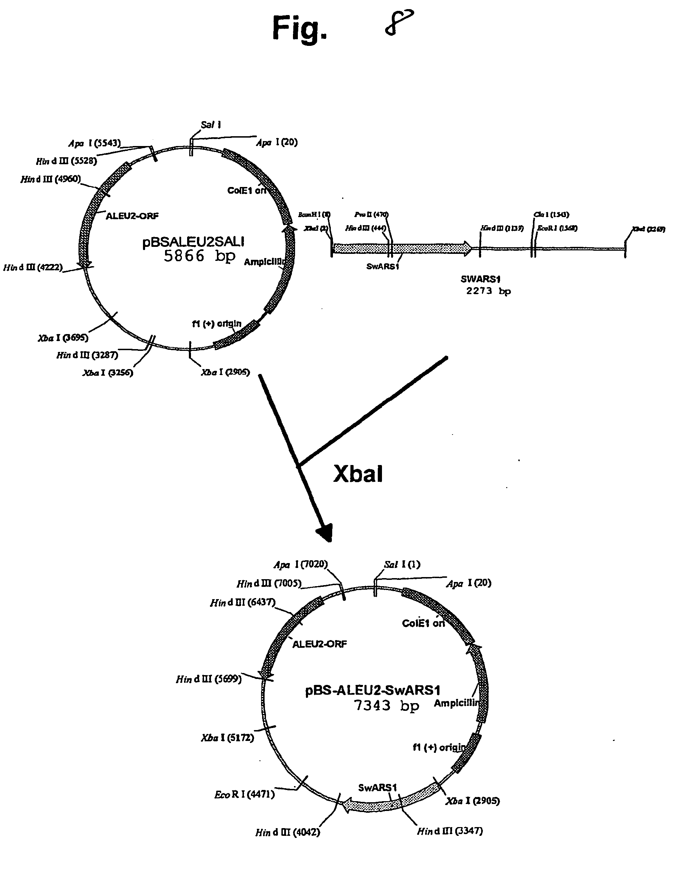

Fig. 8 zeigt das Konstruktionsschema für das Plasmid pBS-ALEU2-SwARS1.

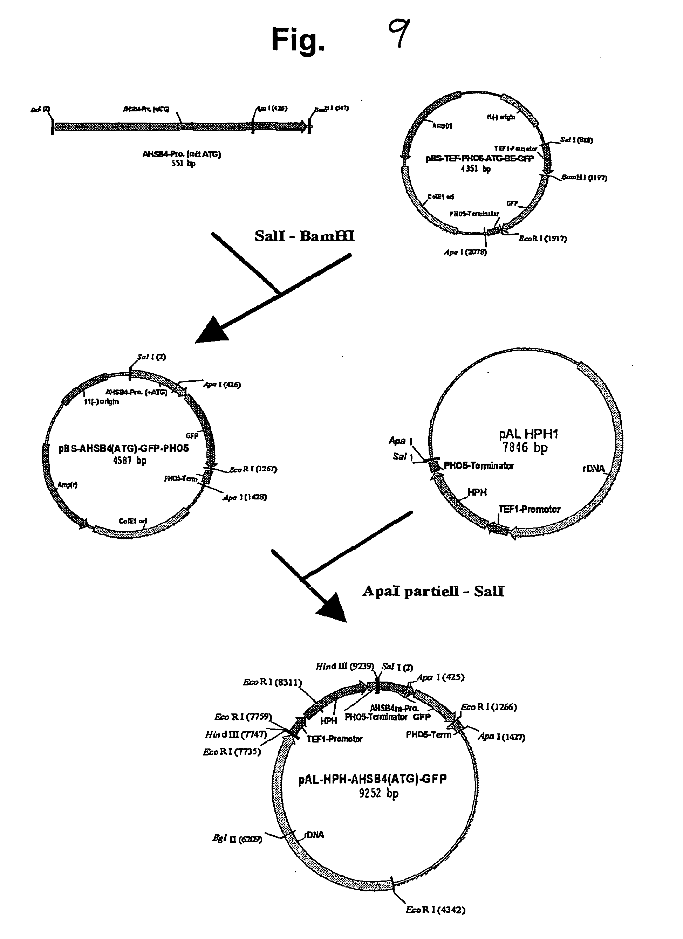

Fig. 9 zeigt das Konstruktionsschema für das Plasmid pAL-HPH-AHSB4(ATG)-GFP.

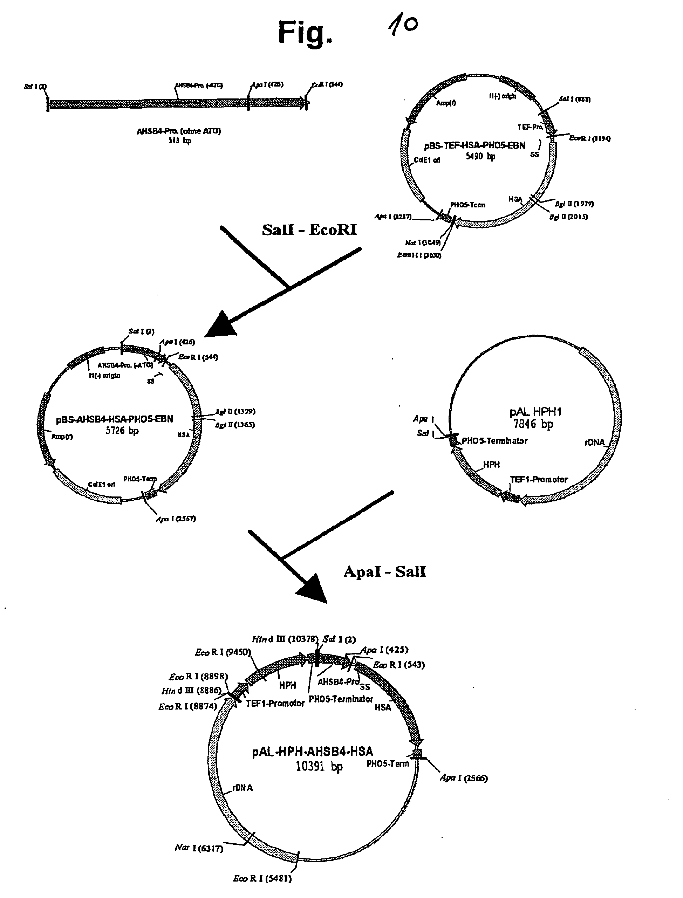

Fig. 10 zeigt das Konstruktionsschema für das Plasmid pAL-HPH-AHSB4-HSA.

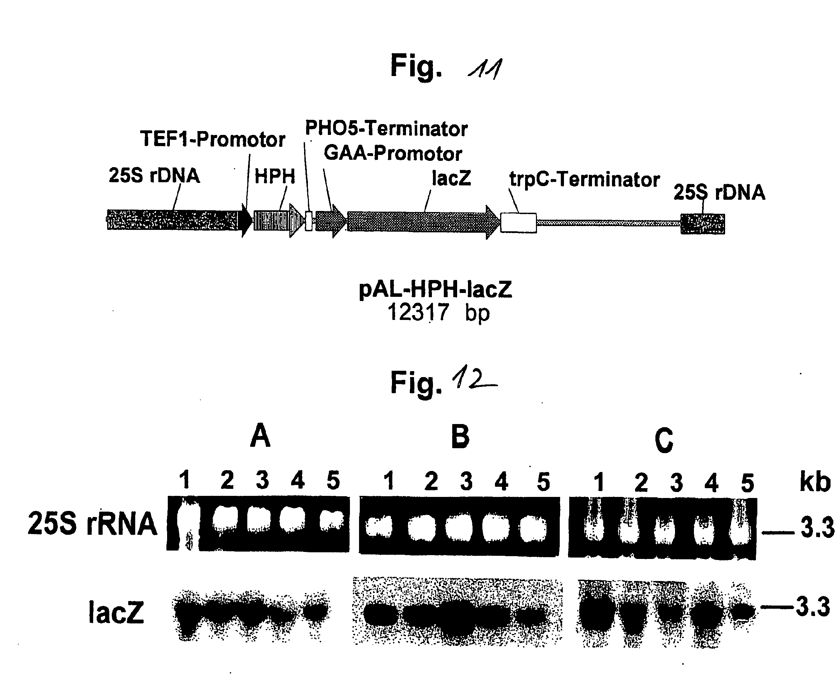

Fig. 11 zeigt die Gen und Restriktionskarte des für die Transformation in *A. adeninivorans* genutzten linearisierten Plasmides pAL-HPH-lacZ.
Fig. 12 zeigt die *lacZ*-Transkriptkonzentration in *A. adeninivorans* LS3/pAL-HPH1-lacZ-80, kultiviert (A) bei 30°C (Hefezellen) bzw. (B) bei 45°C (Myzelien) und 135/pAL-HPH1-lacZ-2, kultiviert (C) bei 30°C (Myzelien). Dazu wurden die Kulturen in HMM mit Maltose als C-Quelle kultiviert, die Zellen nach (1) 20, (2) 36, (3) 45, (4) 60 und (5) 70stündiger Kultivierung geerntet, davon die Gesamt-RNA isoliert und diese für Northern-Hybridisierungen genutzt. Als radioaktiv markierte Probe wurde ein 3 Kbp *Bam*HI/*Ec*oRI-DNA-Fragment mit dem *lacZ*-Gen genutzt.

In den folgenden Beispielen wird die Erfindung näher erläutert.

### Beispiele

### Materialien und Methoden

### Bakterienstämme und Kulturbedingungen

- *E. coli* LE392 (supE supF, hsd R r) (Sambrook et al. 1989).
- *E. coli* TOP F'[mcraΔ (mmr-hsdRMS-mcrBC) F80ΔlacZDM15 DlacX74 deoR recA1 araD139 Δ(ara leu) 7697 galU galK I⁻ rspL andA1 nupG F] - Invitrogen/USA.

Für die Kultivierung der Stämme wurde LB-Medium genutzt (Sambrook et al. 1989).

### Hefestämme und Kulturbedingungen

- *A. adeninivorans* LS3 (Wildtypstamm) (Kunze und Kunze 1994),
- *A. adeninivorans* 135 (Mutante mit verändertem Dimorphismus) (Wartmann et al. 2000)
- *A. adeninivorans* G1211 [*aleu2*] (Samsonova et al. 1996)

Die Hefen wurden in einem komplexen (Vollmedium - YEPD-Medium) bzw. in einen selektiven (Hefeminimalmedium - HMM) Medium mit 1% bzw. 2% Glucose oder Maltose als C-Quelle kultiviert (Rose et al. 1990; Tanaka et al. 1967)

### Transformation von A. adeninivorans

Die Transformationsmethode für *A. adeninivorans* ist ausführlich in Rösel und Kunze (1998) bzw. in Wartmann et al. (1998) beschrieben.

### Northern-Blot-Analyse

Die RNA von *A. adeninivorans* wurde nach der von Stoltenburg et al. (1995) beschriebenen Methode isoliert. Bei der Agarose-Gelelektrophorese wurde nach der von Sambrook et al. (1989) beschriebenen Methode vorgegangen. Die RNA-Hybridisierung wurde bei 42°C nach dem von NEN Research Products/USA mitgelieferten Manual für GeneScreen Nylon-Membranen durchgeführt.

### Southern-Analyse

Die DNA von *A. adeninivorans* wurde nach der von Kunze et al. (1987) beschriebenen Methode präpariert. Die als radioaktive Probe genutzten DNA-Fragmente wurden mit [α-³²P]dATP (Amersham, UK) unter Verwendung eines "Random Primer Labelling Systems" (Gibco, USA) nach den Angaben des Herstellers markiert. Die Hybridisierung erfolgte bei 65°C.

### Western-Blot-Analyse

Zur Westem-Blot-Analyse von GFP, Gaap und lacZp wurde die von Wartmann et al. (2000) beschriebene Prozedur genutzt HSA wurde mit dem Anti-HSA-Antikörper von Biotrend Chemikalien GmbH, BRD nachgewiesen.

### Beispiel 1: Der konstitutive AHSB4-Promotor

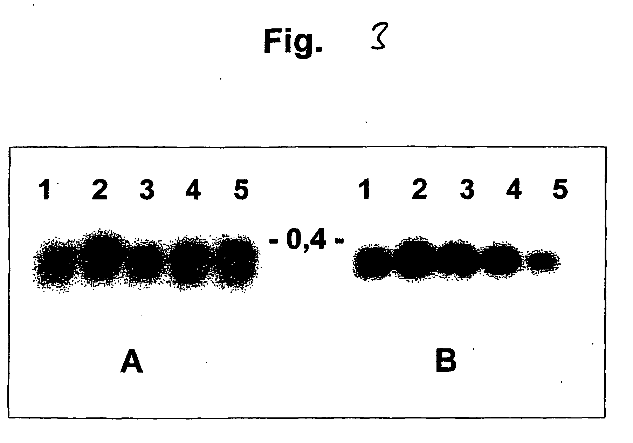

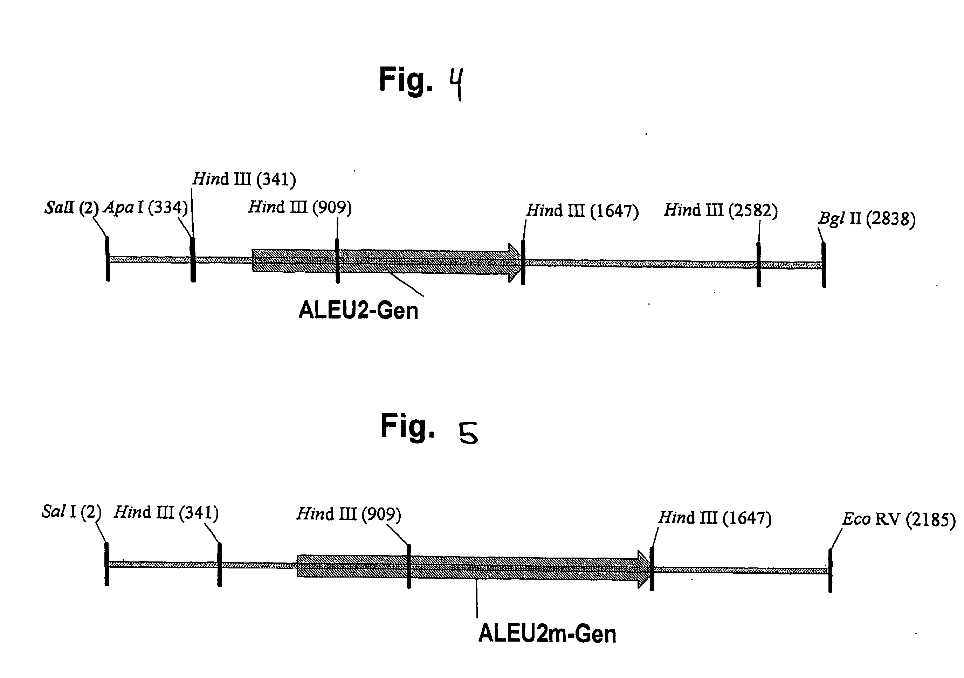

Das *AHSB4*-Gen von *A. adeninivorans,* welches das Histon H4.2 codiert, wurde als 3323 Bp großes *Sal*I/*Sal*I-Fragment isoliert (Fig. 3). Das Gen umfasst einen 360 Nukleotide großen ORF, welcher 120 Aminosäuren kodiert. Dieser ORF wird durch ein 58 Bp großes Intron unterbrochen. Innerhalb der Aminosäuresequenz wurde anhand von Homologie-Vergleichen die H4-Signatur identifiziert. In dem 600 Bp großen Promotorbereich dieses Genes ließen sich eine CAAT-Box (Position -89 - -100) und die TATA-Box (Position -67 - -53) identifizieren (Fig. 4).

Mit Hilfe der Northern-Hybridisierung ließ sich die Aktivität des *AHSB4*-Promotors analysieren. Dazu wurden *A. adeninivorans* LS3 Hefezellen bei 30°C in HMM mit 2% Maltose für 20, 36, 40, 60 und 70 h kultiviert, geerntet und nach Isolation der Gesamt-RNA und Northem-Hybridisierung deren *AHSB4*-Transkript-Akkumulation bestimmt. Dabei konnte eine konstitutive Expression dieses Gens nachgewiesen werden (Fig. 5 A). Auch der Zusatz von 5% Salz zum HMM verändert die *AHSB4*-Transkript-Akkumulation nicht bzw. nur geringfügig (Fig. 5 B).

### Beispiel 2: Konstruktion von Expressionsvektoren zur Herstellung von Protein in Arxula

Als Ausgangsplasmid für die Konstruktion von Expressionsvektoren, die sich für die Herstellung von Protein in einer Hefe der Gattung *Arxula* eignen, wurde das Plasmid pUC19 (Invitrogen, USA) benutzt. In mehreren Klonierungsschritten wurden verschiedene Elemente eingebaut, die für die Selektion der mit den Expressionsvektoren transformierten Wirtsorganismen, zur Erhaltung der eingeschleusten Nukleinsäuren im Wirtsorganismus und zur Expression des für das gewünschte Protein kodierenden Gens geeignet sind.

### 1. Selektionsmarker

Zur Selektion von *Arxula*-Transformanden, die mindestens einen Expressionsvektor enthalten, wurden verschiedene Selektionsmarker benutzt. Zum Einen wurde als Selektionsmarker das aus *E*. *coli* stammende *hph*-Gen benutzt, das eine Selektion von Transformanden anhand ihrer Hygromycin B-Resistenz erlaubt (Rösel und Kunze 1995; Rösel und Kunze 1998). Um eine richtige Transkription des *hph*-Gens in *Arxula* zu gewährleisten, wurde das *hph*-Gen unter die Kontrolle des konstitutiv exprimierten *TEF1*-Promotors von *Arxula adeninivorans* (Rösel und Kunze 1995) gestellt und der *PHO5*-Terminator von *Saccharomyces cerevisiae* (Arima et al. 1983) als Terminator benutzt

Alternativ wurde als Selektionsmarker das *ALEU2*-Gen von *A. adeninivorans* LS3 benutzt. Zur Isolierung dieses Gens wurde *S. cerevisiae* SEY621 0 [leu2] (Robinson et al. 1988) mit einer Plasmidgenbank mit cDNA von *A. adeninivorans* LS3 (YEp112-cDNA) transformiert und die erhaltenen Transformanden wurden nach Komplementierung der *leu2*-Mutation geprüft. Von den erhaltenen LEU2-prototrophen Transformanden wurde die Plasmid-DNA isoliert und diese nach Retransformation in *E. coli* analysiert. Alle analysierten cDNA-Bereiche dieser gescreenten Plasmide waren identisch. Ein 2838 Bp langes *Sal*I/*Bgl*II-Fragment mit dem vollständigen *ALEU2*-Gen (Fig. 36) wurde zwischen der *Sal*I*-* und der *Bgl*II-Schnittstellen des Plasmids pUC19 eingebaut (Plasmid pBSALEUSALI). Die Nukleotidsequenz des *ALEU2*-Gens von *A. adeninivorans* (SEQ ID NO:15) weist eine 78,6%ige Homologie zum *LEU2*-Gen von *S. cerevisiae* auf.

Zur besseren Handhabung des *ALEU2*-Gens als Selektionsmarker wurde die Nukleotidsequenz modifiziert. Mittels Mutagenese wurde der *Apa*I-Restriktionsort so verändert, dass keine Erkennungsstelle mehr für dieses Restriktionsenzym vorhanden ist. Das so modifizierte *ALEU2*-Gen wird im Folgenden *ALEU2m-Gen* bezeichnet. Gleichzeitig wurde das Fragment mit dem *ALEU2m*-Gen auf 2187 Bp verkürzt und mit Hilfe der PCR-Methode mit dem *Sal*I*-* und *Eco*RV-Restriktionsort flankiert (Fig. 37; SEQ ID NO:16).

### 2. Integrations-/Replikationssequenzen für Arxula

Zur Bereitstellung von Expressionsvektoren, die in das Genom einer Hefe der Gattung *Arxula* integriert werden, wurde ein etwa 3,4 Kbp langes *Eco*RI-Fragment der 25S-rDNA von *Arxula adeninivorans* (Rösel und Kunze 1998; Rösel und Kunze 1996) in die *Eco*RI-Schnittstelle des pUC19-Plasmids eingefügt (Plasmid pAL3R). Anschließend wurde das *Eco*RV/*Sal*I-DNA-Fragment mit dem *ALEU2m*-Gen in das 25S-rDNA enthaltende Plasmid pAL3R eingebaut (Fig. 38). Das resultierende Plasmid pAL-ALEU2m lässt sich nach Linearisierung mit *Bgl*II in *A. adeninivorans* nach der von Rösel und Kunze (1998) beschriebenen Methode transformieren. Dazu wurde der Stamm *A. adeninivorans* G1211 [*aleu*2] genutzt. Die erhaltenen Transformanden lassen sich über die Komplementation der *aleu2*-Mutation durch das *ALEU2m*-Gen selektieren.

Die erhaltenen Transformanden wurden charakterisiert. Um die im Genom integrierte Kopiezahl der Plasmide zu bestimmen, wurde die Southem-Hybridisierung genutzt. Dazu wurde von *A. adeninivorans* G1211/pAL-ALEU2m die chromosomale DNA isoliert, mit *Bgl*II restringiert, gelelektrophoretisch getrennt, auf Nitrocellulose geblottet und gegen das radioaktiv markierte pUC19-Plasmid bzw. ein *Bgl*II-Fragment mit dem *ALEU2m*-Gen hybridisiert.

Im Gegensatz zum nicht-transformierten Ausgangsstamm G1211 hybridisiert die DNA der Transformanden G1211/pAL-ALEU2m mit dem Plasmid pUC19, welches der *E. coli*-Bestandteil vom *Arxula*-Vektor pAL-ALEU2m ist. Die radioaktiv markierte Bande hat eine Länge von ca. 8,5 Kbp, d.h. sie ist mit dem Ausgangsplasmid pAL-ALEU2m identisch. Damit wird das Plasmid pAL-ALEU2m in den *Bgl*II-Ort der 25S-rDNA von *A. adeninivorans* integriert.

Zur Bestimmung der ins Genom integrierten Plasmid-Kopiezahl wurde das *Bgl*II-DNA-Fragment mit dem *ALEU2m*-Gen als Hybridisierungsprobe genutzt. Dieses Fragment hybridisiert sowohl mit dem *Arxula* eigenen *ALEU2*-Gen als auch mit dem *ALEU2m*-Gen der Transformanden. In allen Proben (Ausgangsstammm 1211 und Transformanden) ließ sich ein ca. 3 Kbp langes Fragment identifizieren, welches das *Arxula*-eigene *ALEU2*-Gen darstellt (Fig. 39). Im Gegensatz dazu hybridisiert bei der chromosomalen DNA der Transformanden eine weitere 8,5 Kbp Bande, welche das auf Plasmid pAL-ALEU2m lokalisierte *ALEU2m*-Gen umfasst. Werden nun die Intensitäten der jeweiligen *ALEU2*-Bande mit der *ALEU2m*-Bande verglichen, so sind diese gleich. Damit wurde bei allen getesteten Transformanden eine Kopie vom Plasmid pAL-ALEU2m ins *Arxula*-Genom integriert.

Zur Herstellung von Expressionsplasmiden, die in einer Hefe der Gattung *Arxula,* insbesondere *A. adeninivorans* repliziert werden können, wurde die SwARS1-Sequenz von *Schwanniomyces occidentalis* (EMBL-Datenbank, Nr. AJ278886) auf ihre Funktionstüchtigkeit im *Arxula*-System getestet. Dazu wurde das Plasmid pBS-ALEU2-SwARS nach der in Fig. 40 dargestellten Weise konstruiert und in *A. adeninivorans* G1211 [*aleu*2] transformiert. Die Transformanden wurden über ihre Komplementation der *aleu2*-Mutation durch das *ALEU2*-Gen selektiert und anschließend charakterisiert. Zur Konstruktion des entsprechenden Plasmids pBS-ALEU2m-SwARS, das anstelle des *ALEU2*-Gens das modifizierte *ALEU2m*-Gen enthält, wurde das Plasmid pBS-ALEU2-SwARS mit den Restriktionsenzymen *Eco*RI und *Sal*I gespalten. Anschließend wurde das erhaltene *Eco*RI/*Sal*I-Fragment mit der SwARS1-Sequenz mit dem aus der Spaltung des Plasmids pAL-ALEU2m (siehe Fig. 38) mit den gleichen Restriktionsenzymen resultierenden Fragment mit dem ALEU2m-Gen durch Ligation verbunden.

Eine signifikante Steigerung der Transformationsfrequenz gegenüber der integrativen Transformation ließ sich mit den Plasmiden pBS-ALEU2-SwARS und pBS-ALEU2m-SwARS nicht feststellen. Nach der von Rösel und Kunze (1998) beschriebenen Prozedur (ohne vorherige Linearisierung der Plasmid-DNA) wurden 50 bis 200 Transformanden pro µg DNA erhalten. Von diesen Transformanden wurde die Plasmid-DNA isoliert und zur Transformation von *E. coli* verwendet. Im Gegensatz zur integrativen Transformation ließen sich die ARS-enthaltenden Plasmide wieder aus den *Arxula*-Tansformanten isolieren und in *E. coli* retransformieren. Von diesen wurde erneut die Plasmid-DNA isoliert und restringiert. Die dabei erhaltenen Restriktionsmuster waren mit dem des Ausgangsplasmides (pBS-ALEU2-SwARS1 bzw. pBS-ALEU2m-SwARS1) identisch.

Parallel wurden Untersuchungen zur mitotischen Stabilität des Plasmides in *A*. *adeninivorans* G1211 durchgeführt. Dazu wurden die Transformanden für ca. 100 Generationen unter selektiven bzw. nichtselektiven Bedingungen kultiviert und anschließend auf selektiven und nichtselektiven Medien ausgespatelt. Unter diesen Bedingungen werden mitotische Stabilitäten von ca. 90% erreicht.

### 3. Expresionskassette

Die Expressionsvektoren zur Herstellung von Protein in *Arxula* enthalten eine Expressionskassette mit mindestens einem der von der Erfindung bereitgestellten Promotoren und einem Terminator, beispielsweise der *PHO5*-Terminator von *S. cerevisiae.* Zwischen dem jeweiligen Promotor und dem *PHO5*-Terminator ist eine multiple Klonierungsstelle (mit unikalen Schnittstellen für *Eco*RI - *Bam*HI- *Not*I) vorhanden, mit der sich die DNA-Fragmente mit den zur Expression vorgesehenen Genen direkt zwischen dem Promotor und dem PHO5-Terminator einbauen lassen.

### Beispiel 4: Heterologe Genexpression mit Hilfe des AHSB4-Promotors

### 1. Expression des GFP-Gens

Auch eine Expression des *GFP*-Gens mit Hilfe des *AHSB4*-Promotors ist möglich. Dazu wurde der 536 Nukleotide umfassende Promotorbereich (SEQ ID NO:17) einschließlich Startcodon ATG mit den Restriktionserkennungsstellen für *Sal*I und *Bam*HI flankiert. Das über die PCR erhaltene Amplifikationsprodukt musste anschließend "in frame" vor das *GFP*-Gen eingefügt werden. Die dabei erhaltene Kassette mit *AHSB4*-Promotor - *GFP-*Gen - *PHO5*-Terminator konnte mit *Sal*I-*Apa*I aus dem Plasmid pBS-AHSB4(ATG)-GFP-PHO5 wieder herausgespalten und in das *Arxula* Plasmid pAL-HPH1 eingebaut werden (Fig. 48). Das Plasmid pAL-HPH-AHSB4(ATG)-GFP ließ sich nach Linearisierung mit *Bgl*II direkt in *A. adeninivorans* LS3 und 135 transformieren. Die erhaltenen Transformanden wurden über ihre Hygromycin B-Resistenz selektiert.

Zur Überprüfung der GFP-Akkumulation wurde die Fluoreszenzmikroskopie genutzt. Dazu wurden die Transformanden *A*. *adeninivorans* LS3/ pAL-HPH-AHSB4(ATG)-GFP und 135/ pAL-HPH-AHSB4(ATG)-GFP für 48 h in HMM (Tanaka et al. 1967) mit 2% Glucose kultiviert, geerntet und die Zellen am Fluoreszenzmikroskop analysiert.

### 2. Expression des HSA-Gens

Mit Hilfe des *AHSB4*-Promotors konnte auch das *HSA*-Gen in *A. adeninivorans* exprimiert werden. Dazu wurde der 536 Nukleotide umfassende Promotorbereich (SEQ ID NO:17) mit den Restriktionserkennungsstellen für *Sal*I und *Eco*RI flankiert. Das über die PCR erhaltene Amplifikationsprodukt ließ sich direkt vor das *HSA*-Gen einfügen. Die dabei erhaltene Kassette mit *AHSB4*-Promotor- *HSA*-Gen - *PHO5*-Terminator konnte mit *Sal*I-*Apa*I aus dem Plasmid pBS-AHSB4-HSA-PHO5 wieder herausgespalten und in das *Arxula*-Plasmid pAL-HPH1 eingebaut werden (Fig. 49). Das Plasmid pAL-HPH-AHSB4-HSA ließ sich nach Linearisierung mit *Nar*I direkt in *A. adeninivorans* LS3 und 135 transformieren. Die erhaltenen Transformanden wurden über ihre Hygromycin B-Resistenz selektiert.

### Literatur

Altschul SF. et al.(1990) Basic local alignment search tool. J. Mol. Biol. 215: 403-410.
Arima K, Oshima T, Kubotal, Nakamura N, Mizunaga T, Tohe A (1983) The nucleotide sequence of the yeast PHO5 gene: a putative precursor of repressible acid Phosphatase contains a signal peptide. Nucleic Acids Res 11: 1657-1672
Devereux, J. et al. (1984) Nucleic Acids Research 12 (12): 387.
Gellissen G, Hollenberg, CP (1997) Application of yeasts in gene expression studies: a comparison of Saccharomyces cerevisiae, Hansenula polymorpha and Kuyveromyces lactis - a review. Gene 190, 87-97.
Hollenberg CP, Gellissen G (1997) Production of recombinant proteins by methylotrophic yeasts. Curr Opinion Biotechnol 8, 554-560.
Johnston M, Hillier L, Riles L, Albermann K, Andre B, Ansorge W, Benes V, Bruckner M, Delius H, Dubois E, Dusterhoft A, Entian KD, Floeth M, Goeffeau A,Hebling U, Heumann K, Heuss-Neitzel D, Hilbert H, Hilger F, Kleine K, Kotter P, Louis E, Messenguy F, Mewes HW, Hoheisel JD (1997) The nucleotide sequence of Saccharomyces cerevisiae chromosome. Nature 387, 87-90.
Kahana JA, Silver PA (1996) Use of A. victoria green fluorescent protein to study protein dynamics in vivo. Current protocols in molecular biology: 9.6.13.-9.6.19. John Wiley, New York.
Kuchler K, R. Sterne, J Thomer (1989) Saccharomyces cerevisiae STE6 gene product: a novel pathway for protein export in eukaryotic cells. EMBO J 8, 3973-3984.
Kunze G, Bode R, Schmidt H, Samsonova IA, Birnbaum D (1987) Identification of a lys2 mutant of Candida maltosa by means of transformation. Curr Genet 11: 385-391
Kunze G, Kunze I. (1994) Characterization of A. adeninivorans strains from different habitats. Antonie van Leeuwenhoek 65: 607-614.
Kunze G, Kunze I. (1996) Arxula adeninivorans. In: Wolf K (Hrsg.) Nonconventional yeasts. Springer Verlag, Berlin-Heidelberg-New York, S. 389-409.
Kurihara T, Ueda M, Kanayama N, Kondo J, Teranishi Y, Tanaka A (1992) Peroxisomal acetoacetyl-CoA thiolase of an n-alkane- utilizing yeast Candida tropicalis. Eur J Biochem 210, 999-1005.
Middelhoven WJ, Hoogkamer-Te Niet MC, Kreger van Rij NJW (1984) Trichosporon adeninovorans sp. nov., a yeast species utilizing adenine, xanthine, uric acids, putrescine and primary n-alkylamines as the sole source of carbon, nitrogen and energy. Antonie van Leeuwenhoek 50, 369-378.
Needleman und Wunsch (1970) J. Mol. Biol 48: 443-453.
Nishi K, M Yoshida, M Nishimura, M Nishikawa, M Nishiyama, S Horinouchi, T Beppu (1992) A leptomycin resistance gene of Schizosaccharomyces pombe encodes a protein similar to the mammalian P-glycoproteins. Mol Microbiol 6, 761-769.
Ramaswamy NT, Li L, Khalil M, Cannon JF. (1998) Regulation of yeast glycogen metabolism and sporulation by Glc7p protein phosphatase. Genetics 149(1):57-72.
Robinson JS, Klionsky DJ, Banta LM, Emr SD (1988) Protein sorting in Saccharomyces cerevisiae. Isolation of mutants defective in the delivery and processing of multiple vacuolar hydrolases. Mol Cell Biol 8: 4936-4948.
Rose MD, Winston F, Hieter P. (1990) Methods in yeast genetics. A laboratory manual. Cold Spring Harbor Laboratory. Cold Spring Harbor. New York.
Rösel H, Kunze G (1995) Cloning and characterization of a TEF gene for elongation factor 1α from the yeast Arxula adeninivorans. Curr Genet. 28, 360-366.
Rösel H und Kunze G (1996) Identification of a group-I intron within the 25S rDNA from the yeast Arxula adeninivorans. Yeast 12: 1201-1208.
Rösel H, Kunze G (1998) Integrative transformation of the dimorphic yeast Arxula adeninivorans LS3 based on hygromycin B resistance. Curr Genet 33, 157-163.
Sambrook J, Fritsch EF, Maniatis T. (1989) Molecular cloning: a laboratory manual. 2. Ausgabe, Cold Spring Harbor Laboratory, Cold Spring Harbor, New York.
Samsonova IA, Kunze G, Bode R, Böttcher F(1996) A set of genetic markers for the chromosomes of the imperfect yeast A. adeninivorans. Yeast 12: 1209-1217.
Stoltenburg R, Wartmann T, Kunze I, Kunze G. (1995). Reliable method to separate free and membrane-bound polysomes from different species. Bio/Techn 18: 564-568.
Sudbery P (1996) The expression of recombinant proteins in yeasts. Curr Opin Biotechnol 7, 517-524.
Tanaka A, Ohnishi N, Fukui S (1967) Studies on the formation of vitamins and their function in hydrocarbon fermentation. Production of vitamin B6 by Candida albicans in hydrocarbon medium. J Ferment Technol 45, 617-623.
Van der Walt JP, Smith MT, Yamada Y (1990) Arxula gen.nov. (Candidaceae), a new anamorphic, arthroconidial yeast genus. Antonie van Leeuwenhoek 57, 59-61.
Wartmann T, Erdmann J, Kunze I, Kunze G. (2000) Morphology-related effects on gene expression and protein accumulation of the yeast Arxula adeninivorans LS3. Arch. Microbiol. 173: 253-261
Wartmann T, Rösel H, Kunze I, Bode R, Kunze G. (1998) AILV1 gene from the yeast Arxula adeninivorans LS3 - a new selective transformation maker. Yeast 14: 1017-1025

## Patentansprüche

1. Verfahren zum Herstellen von einem oder mehreren Proteinen in einer Wirtszelle der Hefegattung *Arxula,* umfassend:
(i) Klonieren von mindestens einer Nukleinsäure, die ein heterologes Protein kodiert, in einen Expressionsvektor, der einen Promotor des konstitutiv exprimierten AHSB4-Gens aus einer Hefe der Gattung *Arxula* enthält, der die in SEQ ID No : 17 angegebene Sequenz umfasst, so dass die klonierte Nukleinsäure unter der Transkriptionskontrolle des Promotors steht;
(ii) Einführen des in (i) erhaltenen Expressionsvektors in eine zum Herstellen des heterologen Proteins geeignete Wirtszelle der Hefegattung *Arxula;*
(iii) Kultivieren der in (ii) erhaltenen Wirtszelle und ;
(iv) Ernten des Proteins in an sich bekannter Weise.

2. Verfahren nach Anspruch 1, **dadurch gekennzeichnet, dass** der Expressionsvektor weiterhin eine Sequenz umfasst, die die Integration des Vektors oder eines Teils desselben in das Genom der Wirtszelle ermöglicht.

3. Verfahren gemäß einem der Ansprüche 1 bis 2, **dadurch gekennzeichnet, dass** der Expressionsvektor weiterhin eine Sequenz umfasst, die die Replikation des Vektors in der Wirtszelle ermöglicht.

4. Nukleinsäure-Molekül, umfassend:
(1) einen konstitutiven Promotor
der aus folgenden Nukleinsäuren ausgewählt ist
(a) einer Nukleinsäure mit der in SEQ ID NO:17 angegebenen Sequenz;
(b) einer Nukleinsäure mit einer Sequenz, die mindestens 70% Identität mit einer der in (a), angegebenen Sequenzen aufweist;
(c) einer Nukleinsäure, die mit dem Gegenstrang einer der in (a), angegebenen Nukfeinsäuren unter stringenten Bedingungen hybridisiert;
oder
(2) eine Nukleinsäure mit einer Sequenz, die zu der Sequenz einer der in (a) bis (c) angegebenen Nukleinsäuren komplementär ist.

5. Nukleinsäure-Molekül gemäß Anspruch 4, **dadurch gekennzeichnet, dass** die untere (b) angegebene Nukleinsäure mindestens 90% Identität mit einer der in (a), angegebenen Sequenzen oder ihrer Komplementärsequenz aufweist.

6. Nukleinsäure-Molekül gemäß Anspruch 4, **dadurch gekennzeichnet, dass** die unter (b) angegebene Nukleinsäure mindestens 95% Identität mit einer der in (a), angegebenen Sequenzen oder ihrer Komplementärsequenz aufweist.

7. Nukleinsäure-Molekül gemäß einem der Ansprüche 4 bis 6, **dadurch gekennzeichnet, dass** es weiterhin mindestens eine unter der Transkriptionskontrolle des Promotors stehende Nukleinsäuresequenz für ein heterologes Gen umfasst.

8. Expressionsvektor, der mindestens ein Nukleinsäure-Molekül gemäß einem der Ansprüche 4 bis 7 umfasst.

9. Expressionsvektor gemäß Anspruch 8, **dadurch gekennzeichnet, dass** er mindestens ein Markergen enthält.

10. Expressionsvektor gemäß Anspruch 9, **dadurch gekennzeichnet, dass** das Markergen das *ALEU2*-Gen von *Arxula adeninivorans* oder eine Variante desselben ist.

11. Expressionsvektor gemäß Anspruch 10, **dadurch gekennzeichnet, dass** das Markengen eine Sequenz nach SEQ ID NO:15 oder SEQ ID NO:16 umfasst.

12. Expressionsvektor gemäß einem der Ansprüche 8 bis 11, **dadurch gekennzeichnet, dass** er eine Sequenz umfasst, die die Integration des Vektors oder eines Teils desselben in das Genom einer Wirtszelle der Hefegattung *Arxula* ermöglicht.

13. Expressionsvektor gemäß einem der Ansprüche 8 bis 12, **dadurch gekennzeichnet, dass** er eine Sequenz umfasst, die die Replikation des Vektors in einer Wirtszelle der Hefegattung Arxula ermöglicht.

14. Wirtszelle, enthaltend ein Nukleinsäure-Molekül gemäß einem der Ansprüche 4 bis 7 oder einen Expressionsvektor gemäß einem der Ansprüche 8 bis 13.

15. Wirtszelle gemäß Anspruch 14, **dadurch gekennzeichnet, dass** sie der Hefegattung *Arxula* angehört.

16. Wirtszelle gemäß Anspruch 15, **dadurch gekennzeichnet, dass** sie *Arxula adeninivorans* ist.

17. Kit, umfassend:
(a) einen Expressionsvektor gemäß einem der Anspruche 8 bis 13, der zum Klonieren einer Nukleinsäure geeignet ist, welche ein heterologes Protein kodiert, und
(b) eine zur Induktion des Promotors und zum Herstellen des heterologen Proteins geeignete Wirtszelle.

18. Verwendung eines Nukleinsäure-Moleküls gemäß einem der Ansprüche 4 bis 7 oder eines Expressionsvektors gemäß einem der Ansprüche 8 bis 13 oder einer Wirtszelle gemäß einem der Ansprüche 14 bis 16 oder eines Kits gemäß Anspruch 17 zum Expression eines Gens unter der Kontrolle des Promotors.

19. Verwendung eines Nukleinsäure-Moleküls gemäß einem der Ansprüche 4 bis 7 oder eines Expressionsvektors gemäß einem der Ansprüche 8 bis 13 oder einer Wirtszelle gemäß einem der Ansprüche 14 bis 16 oder eines Kits gemäß Anspruch 17 zum Herstellen von einem oder mehreren Proteinen.

## Claims

1. Method for producing one or more proteins in a host cell of the yeast genus *Arxula,* comprising:
(i) cloning of at least one nucleic acid which codes a heterologous protein into an expression vector which contains a promoter of the constitutively expressed AHSB4 gene from a yeast of the genus *Arxula* which comprises the sequence indicated in SEQ ID No: 17, so that the cloned nucleic acid is under the transcription control of the promoter;
(ii) insertion of the expression vector obtained in (i) into a host cell of the yeast genus *Arxula* which is suitable for producing the heterologous protein;
(iii) culturing of the host cell obtained in (ii) and
(iv) harvesting of the protein in a manner known per se.

2. Method according to claim 1, **characterized in that** the expression vector furthermore comprises a sequence which renders possible the integration of the vector or of a part thereof into the genome of the host cell.

3. Method according to one of claims 1 to 2, **characterized in that** the expression vector furthermore comprises a sequence which renders possible the replication of the vector in the host cell.

4. Nucleic acid molecule comprising:
(1) a constitutive promoter which is chosen from the following nucleic acids:
(a) a nucleic acid having the sequence indicated in SEQ ID NO: 17;
(b) a nucleic acid having a sequence which has at least 70 % identity with one of the sequences indicated in (a);
(c) a nucleic acid which hybridizes under stringent conditions with the counter-strand of one of the nucleic acids indicated in (a);
or
(2) a nucleic acid having a sequence which is complementary to the sequence of one of the nucleic acids indicated in (a) to (c).

5. Nucleic acid molecule according to claim 4, **characterized in that** the nucleic acid indicated under (b) has at least 90 % identity with one of the sequences indicated in (a) or its complementary sequence.

6. Nucleic acid molecule according to claim 4, **characterized in that** the nucleic acid indicated under (b) has at least 95 % identity with one of the sequences indicated in (a) or its complementary sequence.

7. Nucleic acid molecule according to one of claims 4 to 6, **characterized in that** it furthermore comprises at least one nucleic acid sequence for a heterologous gene, which is under the transcription control of the promoter.

8. Expression vector which comprises at least one nucleic acid molecule according to one of claims 4 to 7.

9. Expression vector according to claim 8, **characterized in that** it contains at least one marker gene.

10. Expression vector according to claim 9, **characterized in that** the marker gene is the *ALEU2* gene of *Arxula adeninivorans* or a variant thereof.

11. Expression vector according to claim 10, **characterized in that** the marker gene comprises a sequence according to SEQ ID NO: 15 or SEQ ID NO: 16.

12. Expression vector according to one of claims 8 to 11, **characterized in that** it comprises a sequence which renders possible the integration of the vector or of a part thereof into the genome of a host cell of the yeast genus *Arxula.*

13. Expression vector according to one of claims 8 to 12, **characterized in that** it comprises a sequence which renders possible the replication of the vector in a host cell of the yeast genus *Arxula.*

14. Host cell containing a nucleic acid molecule according to one of claims 4 to 7 or an expression vector according to one of claims 8 to 13.

15. Host cell according to claim 14, **characterized in that** it belongs to the yeast genus *Arxula.*

16. Host cell according to claim 15, **characterized in that** it is *Arxula adeninivorans.*

17. Kit comprising:
(a) an expression vector according to one of claims 8 to 13, which is suitable for cloning a nucleic acid which codes a heterologous protein, and
(b) a host cell suitable for induction of the promoter and for production of the heterologous protein.

18. Use of a nucleic acid molecule according to one of claims 4 to 7 or of an expression vector according to one of claims 8 to 13 or of a host cell according to one of claims 14 to 16 or of a kit according to claim 17 for the expression of a gene under the control of the promoter.

19. Use of a nucleic acid molecule according to one of claims 4 to 7 or of an expression vector according to one of claims 8 to 13 or of a host cell according to one of claims 14 to 16 or of a kit according to claim 17 for the production of one or more proteins.

## Revendications

1. Procédé de production d'une ou plusieurs protéines dans une cellule hôte de levure du genre *Arxula,* comprenant les étapes consistant à :
(i) cloner au moins un acide nucléique codant pour une protéine hétérologue dans un vecteur d'expression qui contient un promoteur du gène AHSB4 exprimé constitutivement par une levure du genre *Arxula* et qui comprend la séquence de SEQ ID N° 17 de telle façon que l'acide nucléique cloné se trouve sous le contrôle transcriptionnel du promoteur ;
(ii) introduire le vecteur d'expression obtenu en (i) dans une cellule hôte de levure du genre *Arxula* apte à produire la protéine hétérologue ;
(iii) cultiver la cellule hôte obtenue en (ii), et
(iv) récolter la protéine d'une manière en soi connue.

2. Procédé selon la revendication 1, **caractérisé en ce que** le vecteur d'expression comprend en outre une séquence qui permet l'intégration du vecteur ou d'une partie de celui-ci dans le génome de la cellule hôte.

3. Procédé selon la revendication 1 ou la revendication 2, **caractérisé en ce que** le vecteur d'expression comprend en outre une séquence qui permet la réplication du vecteur dans la cellule hôte.

4. Molécule d'acide nucléique, comprenant :
(1) un promoteur constitutif choisi parmi les acides nucléiques suivants :
(a) un acide nucléique ayant la séquence de SEQ ID N° 17 ;
(b) un acide nucléique ayant une séquence qui présente au moins 70% d'identité avec l'une des séquences indiquées en (a) ;
(c) un acide nucléique qui s'hybride en conditions stringentes avec le brin opposé de l'un des acides nucléiques indiqués en (a) ;
ou
(2) un acide nucléique ayant une séquence complémentaire de l'un des acides nucléiques indiqués en (a) à (c).

5. Molécule d'acide nucléique selon la revendication 4, **caractérisée en ce que** l'acide nucléique indiqué en (b) présente au moins 90% d'identité avec l'une des séquences indiquées en (a) ou avec sa séquence complémentaire.

6. Molécule d'acide nucléique selon la revendication 4, **caractérisée en ce que** l'acide nucléique indiqué en (b) présente au moins 95% d'identité avec l'une des séquences indiquées en (a) ou avec sa séquence complémentaire.

7. Molécule d'acide nucléique selon l'une des revendications 4 à 6, **caractérisée en ce qu'**elle comprend en outre au moins une séquence d'acide nucléique qui se trouve sous le contrôle transcriptionnel du promoteur et code pour un gène hétérologue.

8. Vecteur d'expression, comprenant au moins une molécule d'acide nucléique selon l'une des revendications 4 à 7.

9. Vecteur d'expression selon la revendication 8, **caractérisé en ce qu'**il contient au moins un gène marqueur.

10. Vecteur d'expression selon la revendication 9, **caractérisé en ce que** le gène marqueur est le gène *ALEU2 d'Arxula adeninivorans* ou une variante de celui-ci.

11. Vecteur d'expression selon la revendication 10, **caractérisé en ce que** le gène marqueur comprend une séquence de SEQ ID N° 15 ou de SEQ ID N° 16.

12. Vecteur d'expression selon l'une des revendications 8 à 11, **caractérisé en ce qu'**il comprend une séquence qui permet l'intégration du vecteur ou d'une partie de celui-ci dans le génome d'une cellule hôte de levure du genre *Arxula.*

13. Vecteur d'expression selon l'une des revendications 8 à 12, **caractérisé en ce qu'**il comprend une séquence qui permet la réplication du vecteur dans une cellule hôte de levure du genre *Arxula.*

14. Cellule hôte, contenant une molécule d'acide nucléique selon l'une des revendications 4 à 7 ou un vecteur d'expression selon l'une des revendications 8 à 13.

15. Cellule hôte selon la revendication 14, **caractérisée en ce qu'**elle est une cellule de levure du genre *Arxula.*

16. Cellule hôte selon la revendication 15, **caractérisée en ce qu'**elle est une cellule *d'Arxula adeninivorans.*

17. Kit, comprenant:
(a) un vecteur d'expression selon l'une des revendications 8 à 13, adapté pour le clonage d'un acide nucléique codant pour une protéine hétérologue, et
(b) une cellule hôte adaptée pour l'induction du promoteur et la production de la protéine hétérologue.

18. Utilisation d'une molécule d'acide nucléique selon l'une des revendications 4 à 7 ou d'un vecteur d'expression selon l'une des revendications 8 à 13 ou d'une cellule hôte selon l'une des revendications 14 à 16 ou d'un kit selon la revendication 17 pour l'expression d'un gène sous le contrôle du promoteur.

19. Utilisation d'une molécule d'acide nucléique selon l'une des revendications 4 à 7 ou d'un vecteur d'expression selon l'une des revendications 8 à 13 ou d'une cellule hôte selon l'une des revendications 14 à 16 ou d'un kit selon la revendication 17 pour la production d'une ou plusieurs protéines.
